# EUROPÄISCHE PATENTANMELDUNG

(11) **EP 4 621 793 A1**
(43) Veröffentlichungstag der Anmeldung: **24.09.2025**
(21) Anmeldenummer: 24164204.0
(22) Anmeldetag: 18.03.2024
(51) Int. Cl.: G16H 30/20, G16H 30/40

(54) **TECHNIK ZUM KOMPRIMIEREN MEDIZINISCHER BILDDATEN**

(71) Anmelder: Siemens Healthineers AG, 91301 Forchheim (DE)
(72) Erfinder: Zeller, Mario, 91054 Erlangen (DE)
(74) Vertreter: Siemens Healthineers Patent Attorneys

(57) **Zusammenfassung**

Die Erfindung betrifft eine Technik zum Komprimieren eines digitalen medizinischen Bild-Datensatzes, der mittels eines medizinischen Scanners erhalten wurde. Ein computerimplementiertes Verfahren (100) umfasst einen Schritt des Empfangen (S104), an einer Eingangsschicht eines trainierten Autoencoders, des digitalen medizinischen Bild-Datensatzes von dem medizinischen Scanner. Der trainierte Autoencoder umfasst zumindest eine Extradimension in der Eingangsschicht und optional in einer Ausgangsschicht. Die zumindest eine Extradimension wird insbesondere in Antwort auf einen empfangenen (S102) Hinweis auf mindestens eine Datenredundanzdimension des empfangenen digitalen medizinischen Bild-Datensatzes bereitgestellt. Das Verfahren (100) umfasst ferner einen Schritt des Komprimierens (S106), mittels des trainierten Autoencoders, mindestens eines Teils des empfangenen (S104) digitalen medizinischen Bild-Datensatzes unter Verwendung des empfangenen (S102) Hinweises auf die mindestens eine Datenredundanzdimension und/oder von in der zumindest einen Extradimension empfangenen Daten.

## Beschreibung

Es wird eine Technik zum Komprimieren eines digitalen medizinischen Bild-Datensatzes, der mittels eines medizinischen Scanners erhalten wurde, bereitgestellt. Die Technik umfasst insbesondere ein Verfahren, eine Vorrichtung, ein System, ein Computerprogrammprodukt und ein computerlesbares Medium.

Ein Autoencoder ist ein künstliches neuronales Netz, das dazu genutzt wird, effiziente Kodierungen zu lernen. Das Ziel eines Autoencoders ist es, eine komprimierte Repräsentation (auch: Encoding) für einen Satz Daten zu lernen und somit auch wesentliche Merkmale zu extrahieren. Dadurch kann der Autoencoder zur Dimensionsreduktion genutzt werden. Anwendung haben Autoencoder-Architekturen z.B. in der Bild- oder Videokompression gefunden, wie von Amirhossein Habibian et al. in "Video Compression With Rate-Distortion Autoencoders", arXiv: 1908.05717 [eess.IV], beschrieben, dessen Inhalt hierin durch Bezugnahme aufgenommen ist.

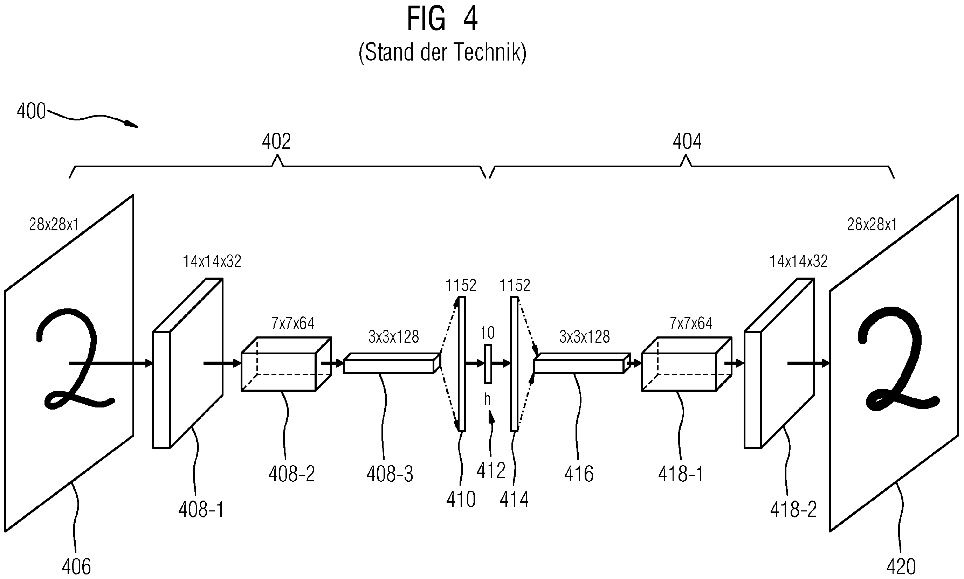

Eine einfache Autoencoderdecoder-Architektur ist in Fig. 4 wiedergegeben entsprechend der Fig. (D) von Christ Kuo/Dr. Dataman, "Convolutional Autoencoders for Image Noise Reduction", Towards Data Science, 20 Nov 2019, dessen Inhalt hierin durch Bezugnahme aufgenommen ist.

Der linke Teil 402 des Autoencoderdecoders in Fig. 4 wird als Encoder, der rechte Teil 404 als Decoder bezeichnet. Im exemplarisch gezeigten Fall der Fig. 4 wird ein Eingangsbild der Größe 28x28 Pixel an der Eingabeschicht 406 des Encoders 402 verwendet (bzw. eingelesen). Danach folgen mehrere faltende Schichten 408-1; 408-2; 408-3, die jeweils eine kleinere Matrix-Größe aber vervielfachte Kanalzahl aufweisen. Anschließend wird die kleinste Matrix an Bezugszeichen 410 in einen eindimensionalen (1D) Vektor umgeschrieben bzw. flachgemacht. Nicht dargestellt in Fig. 4 sind Rectifier Linear Unit (ReLU)-Schichten zwischen den einzelnen faltenden Schichten 408-1; 408-2; 408-3. Die Größe h an Bezugszeichen 412 wird als komprimierte Repräsentation, komprimiertes Bild, Code oder Latent bezeichnet. Im in Fig. 4 gezeigten Fall umfasst das komprimierte Bild 412 nur zehn (10) Werte. Das komprimierte Bild 412 ist später erforderlich, um das finale Bild an der Ausgabeschicht 420 des Decoders 404 auf der rechten Seite der Fig. 4 zu enthalten. Dazu erfolgt der Vorgang nun in umgekehrter Reihenfolge. Der Vektor 414 wird an Bezugszeichen 416 in eine kleinste Matrix-Größe umgewandelt. Danach folgen entfaltende Schichten 418-1; 418-2 und die Ausgabe des dekomprimierten Bilds bzw. finalen Bilds an der Ausgangsschicht 420 des Decoders 404. Der Autoencoderdecoder kann später in Encoder 402 und Decoder 404 aufgeteilt werden, wobei auf die Decoder-Seite 404 später lediglich der Code 412 des jeweiligen Eingangsbildes benötigt wird, um das Ausgangsbild wiederherzustellen.

Für Bildkompressionsaufgaben besteht das Training eines Netzwerkes darin, dass als Eingangs- und Ausgangsbild jeweils das möglichst identische Bild präsentiert werden und als Loss-Funktion z.B. die mittlere quadratische Abweichung (fachsprachlich: Mean Squared Error, MSE) zwischen erzieltem Ausgangsbild und Eingangsbild verwendet wird.

Für zweidimensionale (2D) digitale Kamerabilder ist bekannt, dass ein Rohdatenvolumen, das ein hochauflösendes Bild mit einer großen Anzahl von Sensoren bzw. Pixeln repräsentiert, Redundanzen enthält und ohne wesentlichen Informationsverlust komprimierbar ist. Es ist auch möglich, nur einen geringen Teil der Informationen mittels Sensordaten, im Extremfall mit einer Ein-Pixel-Kamera, aufzunehmen und die Sensordaten anschließend rechnerisch zu einer Abbildung zusammenzusetzen. Diese Beobachtung macht sich z.B. die komprimierte Erfassung (fachsprachlich: Compressed Sensing) zu Nutze.

Medizinische, insbesondere dreidimensionale (3D), Bild-Daten (z.B.

Magnetresonanztomographie (MRT)-Aufnahmen) weisen üblicherweise eine gewisse Datenredundanz auf. Diese kann bei Zeitserien-Aufnahmen wie bei der funktionellen (fMRI) oder dynamischen Bildgebung in der Zeitdimension liegen, bei Multi-Kontrast-Aufnahmen wie der Dixon-Bildgebung oder Mapping-Aufnahmen in der Kontrast-Dimension, in der SchichtDimension oder bei Mehrkanal-Aufnahmen in der Spulen-Dimension.

Es ist daher ein Ziel der vorliegenden Erfindung, eine Lösung für eine Komprimierung von digitalen medizinischen Bild-Datensätzen bereitzustellen. Alternativ oder ergänzend besteht die Aufgabe, eine Technik zum Sparen von Speicherplatz und/oder Ressourcen bei der DatenÜbertragung von digitalen medizinischen Bild-Daten bereitzustellen.

Diese Aufgabe wird gelöst durch ein Verfahren zum Komprimieren eines digitalen medizinischen Bild-Datensatzes, der mittels eines medizinischen Scanners erhalten wurde, durch ein Autoencoder-Netzwerk, durch ein System, durch ein Computerprogramm bzw. Computerprogrammprodukt und durch ein computerlesbares Speichermedium gemäß den beigefügten unabhängigen Ansprüchen. Vorteilhafte Aspekte, Merkmale und Ausführungsformen sind in den abhängigen Ansprüchen und in der folgenden Beschreibung zusammen mit Vorteilen beschrieben.

Im Folgenden wird die erfindungsgemäße Lösung in Bezug auf das beanspruchte Verfahren zum Komprimieren eines digitalen medizinischen Bild-Datensatzes, der mittels eines medizinischen Scanners erhalten wurde, sowie in Bezug auf das beanspruchte Autoencoder-Netzwerk (kurz: Autoencoder) beschrieben. Merkmale, Vorteile oder alternative Ausführungsformen hierin können den anderen beanspruchten Gegenständen (z.B. dem System umfassend das Autoencoder-Netzwerk, dem Computerprogramm oder einem Computerprogrammprodukt) zugeordnet werden und umgekehrt. Mit anderen Worten: Die Ansprüche für das Autoencoder-Netzwerk und/oder das System umfassend das Autoencoder-Netzwerk können durch Merkmale verbessert werden, die im Zusammenhang mit dem Verfahren beschrieben oder beansprucht werden. In diesem Fall werden die funktionalen Merkmale des Verfahrens durch Struktureinheiten des Autoencoder-Netzwerks und/oder des Systems verkörpert und umgekehrt.

Gemäß einem Verfahrensaspekt wird ein (insbesondere computer-implementiertes) Verfahren zum Komprimieren eines digitalen medizinischen Bild-Datensatzes, der mittels eines medizinischen Scanners erhalten wurde, bereitgestellt. Das Verfahren umfasst den Schritt des Empfangens einen Hinweis auf mindestens eine Datenredundanzdimension hinsichtlich eines zu empfangenden digitalen medizinischen Bild-Datensatzes. Das Verfahren kann ferner einen Schritt des Auswählens eines trainierten Autoencoders in Antwort auf den empfangenen Hinweis umfassen.

Das Verfahren umfasst ferner den Schritt des Empfangens des digitalen medizinischen Bild-Datensatzes von dem medizinischen Scanner an einer Eingangsschicht des (oder eines) trainierten Autoencoders. Der trainierte Autoencoder kann zumindest eine Extradimension in der Eingangsschicht umfassen. Optional umfasst der trainierte Autoencoder ferner zumindest eine Extradimension in einer Ausgangsschicht. Die zumindest eine Extradimension (insbesondere in der Eingangsschicht) kann dazu ausgebildet sein, einen Hinweis auf mindestens eine Datenredundanzdimension des empfangenen digitalen medizinischen Bild-Datensatzes zu empfangen. Alternativ oder ergänzend kann die Extradimension (in der Eingangsschicht und/oder optional in der Ausgangsschicht) in Antwort auf einen empfangenen Hinweis auf mindestens eine Datenredundanzdimension des empfangenen digitalen medizinischen Bild-Datensatzes bereitgestellt werden. Weiterhin alternativ oder ergänzend kann die zumindest eine Extradimension in der Eingangsschicht zum Empfangen von Daten im Hinblick auf die Datenredundanzdimension ausgebildet sein. Vorzugsweise können die an der zumindest einen Extradimension in der Eingangsschicht empfangenen Daten zumindest einen, insbesondere im Hinblick auf die Datenredundanzdimension ausgewählten, Teil des empfangenen digitalen medizinischen Bild-Datensatzes umfassen.

Der Hinweis auf die mindestens eine Datenredundanzdimension des empfangenen digitalen medizinischen Bild-Datensatzes kann implizit sein und/oder implizit empfangen werden (z.B. kann der trainierte Autoencoder für eine ausgewählte Datenredundanzdimension ausgewählt und/oder angesteuert werden). Implizit sein oder implizit empfangen werden meint hier implizit mit den anderen in der Eingangsschicht empfangenen Daten empfangen werden. So kann z.B. der Hinweis aus dem empfangenen digitalen medizinischen Bild-Datensatzes oder einem Teil dessen abgeleitet werden und damit ist der Hinweis implizit bereits in den empfangenen Daten enthalten. Alternativ oder kumulativ ist es möglich, dass ein Anwender über eine Benutzerschnittstelle den Hinweis eingibt oder über ein Feld auf der Benutzeroberfläche auswählt.

Das Verfahren umfasst ferner den Schritt des Komprimierens mindestens eines Teils des empfangenen digitalen medizinischen Bild-Datensatzes mittels des trainierten Autoencoders unter Verwendung des empfangenen Hinweises auf die mindestens eine Datenredundanzdimension und/oder unter Verwendung von an der zumindest einen Extradimension empfangenen Daten (z.B. umfassend mindestens eine teilweise Kopie des digitalen medizinischen Bild-Datensatzes, insbesondere umfassend benachbarte Schichten und/oder zeitliche Instanzen einer mehrschichtigen und/oder Zeitserien-Aufnahme beispielsweise für eine Schichtdimension bzw. eine Zeitdimension als Datenredundanzdimension).

Mittels der erfindungsgemäßen Technik zum Komprimieren eines digitalen medizinischen Bild-Datensatzes kann Speicherplatz für (insbesondere herkömmlicherweise umfangreiche) digitale medizinische Bild-Daten eingespart werden. Alternativ oder ergänzend kann die erfindungsgemäße Technik zum Komprimieren eines digitalen medizinischen Bild-Datensatzes eine ressourcensparende, zeitnahe, vollständige und/oder fehlerfreie Übertragung digitaler Bild-Daten ermöglichen, insbesondere bei geringer Übertragungsbandbreite und/oder geringer Übertragungsgeschwindigkeit.

Der medizinische Scanner (kurz: Scanner) kann auch als medizinischer bildgebender Scanner und/oder als medizinisches bildgebendes System bezeichnet werden. Alternativ oder ergänzend kann der medizinische Scanner einen Magnetreseonanzthomographen (MRT), einen Computertomographen (CT), einen Positronen-Emissions-Tomographen (PET), einen Einzelphotonen-Emissions-Computertomographen (fachsprachlich: Single Photon Emission Computed Tomography, SPECT) oder eine Kombination umfassen (z.B. PET/CT oder SPECT/CT).

Der MRT kann auch als MRT-Scanner bezeichnet werden.

In einem Ausführungsbeispiel kann der trainierte Autoencoder passend zum empfangenen Hinweis auf die mindestens eine Datenredundanzdimension ausgewählt werden, beispielsweise von einer vorgeschalteten und/oder übergeordneten Stelle. Die vorgeschaltete und/oder übergeordnete Stelle kann eine digitale Verarbeitungseinheit sein und den passenden Autoencoder, der für die Verarbeitung des empfangenen digitalen medizinischen Bild-Datensatzes durch Ausnutzung einer Datenredundanz (z.B. in einer bestimmten Richtung) besonders gut geeignet ist, auswählen. In der MRT-Bildgebung liegt insbesondere in der Spulendimension üblicherweise eine (zumindest gewisse) Datenredundanz vor (insbesondere werden heutzutage immer mehrere Spulenkanäle aufgenommen), weswegen schon ein auf die Spulendimension optimierter Autoencoder bei allen MRT-Bild-Datensätzen zu einer Datenreduktion führen kann, auch wenn für bestimmte Anwendungsfälle (z.B. Datenredundanz in Zeitrichtung) ein spezieller Autoencoder noch bessere Ergebnisse liefern kann.

Die mindestens eine Extradimension in der Eingangsschicht kann mindestens ein zusätzliches Neuron sein, das (oder die) insbesondere zusätzlich zu den Neuronen der Eingangsschicht eines herkömmlichen Autoencoders bereitgestellt wird.

Alternativ oder ergänzend kann die Eingangsschicht in mindestens zwei Unterschichten unterteilbar sein. Beispielsweise kann eine erste Unterschicht (auch: Eingangsschnittstelle) zum (z.B. konventionellen) Empfangen des digitalen medizinischen Bild-Datensatzes ausgebildet sein (und/oder die konventionellen Eingangsneuronen des Autoencoders umfassen). Eine zweite Unterschicht kann die mindestens eine Extradimension (und/oder das oder die zusätzlichen Neuronen) umfassen. Beispielsweise kann die zweite Unterschicht Teile (z.B. Schichten und/oder zeitliche Instanzen einer Zeitserienaufnahme) des digitalen medizinischen Bild-Datensatzes umsortieren, vervielfachen und/oder bündeln. Die Verarbeitung des digitalen medizinischen Bild-Datensatzes unter Verwendung der Datenredundanzdimension kann dann, insbesondere basierend auf dem Umsortieren, Vervielfachen und/oder Bündeln in der zweiten Unterschicht, in nachfolgenden verborgenen Schichten des Autoencoders erfolgen.

Die Datenredundanzdimension (kurz: Redundanzdimension) kann (insbesondere mindestens) eine weitere Dimension der Eingangsschicht des trainierten Autoencoders sein, über die redundante Daten erfasst werden. Die Datenredundanz (kurz: Redundanz) kann - je nach Anwendung - in unterschiedlichen technischen Parametern oder Aspekten begründet sein. So kann die Datenredundanz z.B. spezifisch sein für eine Art einer Aufnahme, z.B. hinsichtlich einer Modalität des medizinischen Scanners, eines Aufnahmeprotokolls (auch: Bildgebungsprotokoll) und/oder einer zugrundeliegenden medizinischen Fragestellung. Alternativ oder ergänzend kann die Datenredundanz bzw. damit die Datenredundanzdimension insbesondere eine Zeitdimension (z.B. bei einer Aufnahme über mehrere Herzzyklen und/oder Atemzyklen), eine Schichtdimension (z.B. bei einer aus Schichten zusammengesetzten 3D Aufnahme), eine Kontrastdimension (z.B. bei einer Multikontrastaufnahme) und/oder eine Spulendimension (z.B. bei einer mehrkanaligen MRT-Aufnahme) umfassen. Beispielsweise kann die Datenredundanz bzw. die Datenredundanzdimension einem Bildgebungsprotokoll (z.B. umfassend mehrkanalige Spulen) und/oder zumindest einem Akquisitionsparameter zugeordnet sein. Alternativ oder ergänzend kann die Datenredundanz bzw. die Datenredundanzdimension durch eine überlappende Bildgebung (insbesondere in überlappenden anatomischen Bereichen), durch mehrfache Aufnahmen bzw. wiederholte Messungen, durch Verwendung von Mehrfachspulensystemen und/oder durch parallele Messungen verursacht werden.

Der Hinweis auf mindestens eine Datenredundanzdimension kann aus dem empfangenem digitalen medizinischen Bild-Datensatz berechnet werden. Der Hinweis kann alternativ oder ergänzend aus Metadaten berechnet werden. Die Metadaten können die Art der medizinischen Fragestellung, die Modalität, den zumindest einen Akquisitionsparameter, Daten in Bezug auf das Aufnahmeprotokoll und/oder Daten in Bezug auf den Scanner umfassen. Alternativ oder ergänzend kann der Hinweis auf mindestens eine Datenredundanzdimension implizit sein. Z.B. kann der Autoencoder aus dem empfangenem digitalen medizinischen Bild-Datensatz und/oder aus den Metadaten die Datenredundanzdimension deduzieren bzw. herleiten.

Der digitale medizinische Bild-Datensatz (im Folgenden auch kurz: medizinische Bild-Datensatz) kann Rohdaten umfassen, die z.B. mittels eines MRT-Scans erhalten wurden, insbesondere k-Raum-Daten. Alternativ oder ergänzend kann der medizinische Bild-Datensatz vorverarbeitete und/oder (z.B. Fourier-) transformierte Daten umfassen, die auf Rohdaten basieren. Der medizinische Bild-Datensatz kann alternativ oder ergänzend rekonstruierte Bilder umfassen. Der medizinische Bild-Datensatz kann direkt von einem MRT-Scanner erhalten werden. Alternativ kann der medizinische Bild-Datensatz aus einem Speicher eingelesen werden.

Der trainierte Autoencoder kann einen Encoder eines trainierten Autoencoderdecoders umfassen. Eine Netzwerk-Architektur des Encoders und des Decoders kann unabhängig voneinander sein. Alternativ oder ergänzend kann in einem Ausführungsbeispiel der Decoder eine inverse Schicht- (auch: Layer-) Architektur des Encoders umfassen.

Ein Autoencoder (kurz: Encoder) kann eine Eingangsschicht umfassen. Die Eingangsschicht kann dazu ausgebildet sein, einen (oder den) digitalen medizinischen Bild-Datensatz, der vom medizinischen Scanner (z.B. MRT) aufgenommen wurde, (oder eine vorverarbeitete und/oder transformierte Variante des digitalen medizinischen Bild-Datensatzes) zu empfangen.

Die zumindest eine Extradimension in der Eingangsschicht kann dazu ausgebildet sein, die Datenredundanz (insbesondere in der Datenredundanzdimension) auszunutzen. Bei einer Aufnahme von 64 Repetitionen mit z.B. 256x256-Matrizen werden beispielsweise nicht jede der 256x256-Matrizen nacheinander in den Autoencoder eingespeist (und/oder geschickt), sondern ein Block aus 256x256x64. Die Repetitionsdimension stellt also in diesem Beispiel die Extradimension dar.

Der Autoencoder kann ferner eine Mehrzahl faltender Schichten (fachsprachlich: Convolutional Layers) umfassen. Alternativ oder ergänzend kann der Autoencoder eine oder mehrere Rectified Linear Units (ReLU) und/oder Max-Pooling Layers umfassen.

Der Autoencoder kann ferner eine (z.B. Ausgangs-) Schicht (z.B. eine Fully Connected Layer) umfassen, die eine komprimierte Repräsentation (auch: komprimierte Version, Code oder Latent) des medizinischen Bild-Datensatzes bereitstellt.

Der Begriff "Komprimieren" ist im Sinne der Erzeugung einer effizienten Repräsentation des digitalen medizinischen Bild-Datensatzes zu verstehen. Die Erzeugung einer effizienten Repräsentation kann eine reine Datenkompression bedeuten. Alternativ oder ergänzend kann die Erzeugung einer effizienten Repräsentation eine Datenrekonstruktion und/oder eine Dimensionalitätsreduktion umfassen.

Das Komprimieren (auch: die Kompression) kann auch als Kodieren (fachsprachlich: Encoding) bezeichnet werden. Das Ergebnis des Komprimierens kann eine komprimierte Darstellung (auch: Repräsentation) der medizinischen Bild-Daten sein.

Das Komprimieren kann verlustbehaftet sein. Das Komprimieren kann z.B. ein Speichern eines Bruchteils (z.B. ein Zehntel des Speicherplatzes) des (insbesondere ursprünglich) empfangenen digitalen medizinischen Bild-Datensatzes umfassen (z.B. von 800MB nach Komprimieren und/oder Kodieren statt 8GB vor Komprimieren und/oder Kodieren).

Die komprimierte Version des medizinischen Bild-Datensatzes kann die Komprimierung mindestens eines Teils des empfangenen digitalen medizinischen Bild-Datensatzes umfassen. Beispielsweise kann der mindestens eine Teil des empfangenen digitalen medizinischen Bild-Datensatzes nur einen Teil des k-Raums einer MRT-Aufnahme umfassen, für den die Komprimierung ausgeführt wird.

Die komprimierte Version des medizinischen Bild-Datensatzes kann einem Autodecoder (kurz: Decoder) bereitgestellt werden (z.B. an einer Eingangsschicht, insbesondere einer Fully-Connected Layer).

Der Autodecoder kann eine Mehrzahl entfaltender Schichten (fachsprachlich: Deconvolutional Layers) umfassen. Alternativ oder ergänzend kann der Autodecoder eine oder mehrere ReLUs und/oder Max Depooling Layers umfassen.

Der Autodecoder kann ferner eine Ausgangsschicht (fachsprachlich: Output Layer) umfassen. Die Ausgangsschicht kann dazu ausgebildet sein, einen dekomprimierten digitalen medizinischen Bild-Datensatz bereitzustellen. Der dekomprimierte digitale medizinische Bild-Datensatz kann eine Dekomprimierung der komprimierten Version des medizinischen Bild-Datensatzes umfassen.

Die Dekomprimierung und der ursprüngliche medizinische Bild-Datensatz stimmen vorzugsweise in wesentlichen Merkmalen überein (z.B. hinsichtlich Art und Lage anatomischer Strukturen, und/oder hinsichtlich Vorhandensein, Lage und Größe einer Abnormalität, insbesondere einer Läsion und/oder eines Tumors).

Der Autoencoder und der Autodecoder können gemeinsam trainiert sein, um eine Ähnlichkeit zwischen dem (insbesondere ursprünglich) empfangenen digitalen medizinischen Bild-Datensatz und dem dekomprimierten medizinischen Bild-Datensatz zu maximieren. Beispielsweise kann eine Verlustfunktion (fachsprachlich: Loss Function) eine mittlere quadratische Abweichung (fachsprachlich: Mean Squared Error, MSE; und/oder - möglicherweise bis auf Gesamtnormierung - äquivalent zum L2-Loss), eine mittlere absolute Abweichung (fachsprachlich: Mean Absolute Error, MSE; und/oder - möglicherweise bis auf Gesamtnormierung - äquivalent zum L1-Loss) und/oder einen Index struktureller Ähnlichkeit (fachsprachlich: Structural Similarity Index Measure, SSIM) umfassen. Die MSE, MAE und/oder der SSIM kann pro Voxel (und/oder pro Pixel) bestimmt werden. Alternativ oder ergänzend kann die Verlustfunktion einen Mittelwert der MSE, MAE und/oder des SSIM pro Voxel (und/oder pro Pixel) umfassen.

Der Autoencoder (und optional der Autodecoder) kann auf unterschiedliche Arten trainiert sein und/oder in verschiedenen Ausführungsbeispielen die mindestens eine Extradimension (insbesondere in der Eingangsschicht und optional in der Ausgangsschicht) realisieren. Die verschiedenen Ausführungsbeispiel und/oder Trainingsarten können miteinander kombinierbar sein.

Gemäß einem ersten Ausführungsbeispiel kann der Autoencoder (und optional der Autodecoder) auf eine oder mehrere maximal mögliche Datenredundanzdimensionen trainiert werden. Bei einer Schichtdimension kann die maximale Zahl z.B. 256 sein. Wenn es weniger Schichten zu komprimieren gibt, z.B. nur 30, können z.B. einzelne Schichten, die Nullen enthalten, (insbesondere künstlich) hinzugefügt werden.

In einem zweiten Ausführungsbeispiel kann der Autoencoder (und optional der Autodecoder) auf eine vorbestimmte (und/oder "typische") Schichtanzahl trainiert werden, z.B. 30 Schichten. Wenn es dann z.B. 60 Schichten in dem empfangenen digitalen medizinischen Bild-Datensatz gibt, kann der Eingangsdatensatz passend zerschnitten werden, im einfachsten Fall in zwei Blöcke ä ja 30 Schichten.

In einem dritten Ausführungsbeispiel können in einem (insbesondere komplexeren) Fall die Blöcke des Autoencoder (und optional des Autodecoders) eine teilweise (und/oder gewisse) Überlappung aufweisen (z.B. "Sliding Window"). Hierdurch kann eine Bildung von sichtbaren Übergängen (insbesondere an den Rändern z.B. des digitalen medizinischen Bild-Datensatzes) vermieden werden. Z.B. kann ein Überlapp von drei (3) Schichten gebildet werden.

In einem vierten Ausführungsbeispiel kann werden in der Eingangsschicht, um mehr Informationen über die Datenredundanzen zu lernen, auch eine benachbarte Matrix mit übermittelt werden (z.B. die zwei Nachbarschichten der zu komprimierenden Schicht). Ressourcen können dann gespart werden, indem die Komprimierung des digitalen medizinischen Bild-Datensatzes (auch: der Latent-Vektor) kleiner gewählt werden kann, da mehr Datenredundanzen erkannt werden können bzw. da bei gleicher Zahl an Ausgangsdatensätzen eine Mittelung der in mehreren Übertragungsvorgängen an unterschiedlichen Positionen übermittelten gleichen Matrix erfolgenden kann.

Das Verfahren kann ferner einen Schritt des Übertragens des komprimierten digitalen medizinischen Bild-Datensatzes umfassen. Alternativ oder ergänzend kann das Verfahren ferner einen Schritt des Speicherns des komprimierten digitalen medizinischen Bild-Datensatzes umfassen.

Der komprimierte digitale medizinische Bild-Datensatz kann an eine Cloud, eine vom medizinischen Scanner (z.B. MRT) räumlich getrennte Rechenvorrichtung (und/oder einen ausgelagerten Rechencluster) und/oder einen (insbesondere zentralen) Speicher (z.B. einer Radiologie-Abteilung, einer medizinischen Einrichtung und/oder einer Datenbank für elektronische Patientenakten, fachsprachlich: Electronic Health Records, EHRs) übertragen werden. Beispielsweise kann eine Bild-Rekonstruktion Cloud-basiert ausgeführt werden.

Die Übertragung des komprimierten digitalen medizinischen Bild-Datensatz kann ressourcenschonend (z.B. hinsichtlich Bandbreite und/oder Zeit) und/oder zuverlässig sein, z.B. bei einer langsamen (insbesondere drahtgebundenen und/oder drahtlosen) Internetverbindung.

Alternativ oder ergänzend kann der komprimierte digitale medizinische Bild-Datensatz (z.B. nach Übertragen) gespeichert werden, z.B. in dem (insbesondere zentralen) Speicher (und/oder in iner Cloud). Aufgrund der Komprimierung kann ein Vielfaches (z.B. um einen Faktor zehn) im Vergleich zum herkömmlichen Speicherplatz eingespart werden. Dadurch kann eine Nutzung der Speicherkapazitäten optimiert werden.

Der empfangene Hinweis auf die mindestens eine Datenredundanzdimension kann eine Information (z.B. umfassend ein bis mehrere Bits) sein hinsichtlich einer Art einer Datenredundanz. Alternativ oder ergänzend kann der empfangene Hinweis eine Auswahl (und/oder ein Aktivierungsbefehl), insbesondere hinsichtlich der zumindest einen Extradimension, des trainierten Autoencoders aus einer Menge trainierter Autoencoder sein, wobei jeder Autoencoder aus der Menge trainierter Autoencoder für eine vorbestimmte (insbesondere unterschiedliche) Art von Datenredundanz trainiert ist. Weiterhin alternativ oder ergänzend kann der empfangene Hinweis eine Kopie zumindest eines Teils des digitalen medizinischen Bild-Datensatzes sein (der insbesondere vom herkömmlichen Teil der Eingangsschicht empfangen wird).

Der empfangene Hinweis auf die mindestens eine Datenredundanzdimension (kurz: Hinweis) kann anzeigen, welche Datendimension vermutlich redundant ist (z.B. Spulenkanäle, Zeit, 2D Schichten und/oder anatomische Region). Der Hinweis kann insbesondere aus Metadaten algorithmisch berechnet bzw. abgeleitet werden (z.B. aus den Bild-Daten, aus der Modalität, aus dem Bildgebungsprotokoll und/oder aus einer der Bildgebung zugrundeliegenden medizinischen Fragestellung).

In einer Ausführungsform kann der Hinweis über die zumindest eine Extradimension (kurz: die Extradimension) des Autoencoders erfasst werden. Die Extradimension dient in dieser Ausführungsform also zum Erfassen des Hinweises. Damit kann die Komprimierung getunt und/oder an- und abgestellt werden. Insbesondere kann ein umfassendes Modell (und/oder ein Autoencoder mit einer Vielzahl, insbesondere verborgener, Schichten und/oder Neuronen) trainiert werden für unterschiedliche Komprimierungsszenarien.

In einer weiteren Ausführungsform kann der Hinweis die Architektur des Autoencoders definieren. Der Hinweis dient in dieser Ausführungsform nur dazu, den passenden (und/oder passend trainierten) Autoencoder aus einer Datenbank von mehrere Autoencodern, die für unterschiedliche Anwendungsfälle trainiert sind, auszuwählen. In der Datenbank kann ein "kleiner, spezieller" Autoencoder z.B. für Kardio-MRT (insbesondere mit Datenredundanz in der Zeitdimension) und/oder für Multikontrast-MRT (insbesondere mit Datenredundanz in der Kontrastdimension) in trainierter Form gespeichert sein. Der "kleine, spezielle" Autoencoder wird in der Inferenz nur ausgewählt. Im dieser Ausführungsform können verschiedene kleine Autoencoder modular und jeweils einfacher zu trainieren sein.

Der Hinweis bzw. die in der Extradimension (insbesondere der Eingangsschicht) erfassten Daten können eine (insbesondere ressourcensparende) Information (z.B. "Multikontrast MRT", insbesondere ausgedrückt durch eine kurze Bitsequenz) oder die (z.B. Multikontrast MRT) Bild-Daten selbst umfassen.

Die Datenredundanz entlang der Datenredundanzdimension kann ausgenutzt werden, indem sich z.B. in einer Kardio-Zeitserie hauptsächlich die Bildinformation in der Umgebung des Herzens verändert, während die Bildinformation am Rande im Wesentlichen statisch bleibt.

Die zumindest eine Extradimension (kurz: die Extradimension) in der Ausgangsschicht kann dazu ausgebildet sein, einem (insbesondere mit dem Autoencoder gemeinsam trainierten) Autodecoder eine Information hinsichtlich des verwendeten empfangenen Hinweises im Schritt des Komprimierens bereitzustellen. Beispielsweise kann der komprimierte digitale medizinische Bild-Datensatz ein oder mehrere Bits (insbesondere an einer vorbestimmten Stelle einer Bitsequenz) umfassen, die die Art der Datenredundanzdimension kodieren. Das ein oder die mehreren Bits, die die Art der Datenredundanzdimension kodieren, können mit dem komprimierten digitalen medizinischen Bild-Datensatz konkateniert sein.

Auf die Extradimension in der Ausgangsschicht des Autoencoders kann in einigen Ausführungsformen verzichtet werden. Z.B. können bei Mehrschichtaufnahmen Informationen aus Nachbarschichten genutzt werden, um eine bessere Komprimierbarkeit zu erreichen. Bereiche, in denen sich auch in Nachbarschichten ähnliche Texturen oder einfach nur Rauschen finden, können dann stärker komprimiert werden. Ein mit dem Autoencoder gemeinsam trainiert Autodecoder kann die nötige Information über die Datenredundanzdimension als implizit erfüllt annehmen, wenn er den komprimierten digitalen medizinischen Bild-Datensatz empfängt.

Das Verfahren kann ferner einen Schritt des Bestimmens eines Kompressionsfaktors für das Komprimieren des mindestens einen Teils des empfangenen digitalen medizinischen Bild-Datensatzes umfassen.

Der Kompressionsfaktor kann für eine flexible Netzwerk-Architektur des Autoencoders (und/oder des Autodecoders) einstellbar sein. Der Autoencoder und der Autodecoder können für jeweils den gleichen Kompressionsfaktor und Dekompressionsfaktor trainiert sein. Die Flexibilität des Bestimmens (auch: Festlegens) des Kompressionsfaktors kann vorteilhafterweise abhängig von einer Art der medizinischen (z.B. MRT) Aufnahme sein. In einem Ausführungsbeispiel kann beispielsweise ein Autoencoder für eine Vielzahl MRT-Aufnahmen gemäß unterschiedlichen Bildgebungsprotokollen trainiert sein.

Der medizinische Scanner kann als Modalität MRT, CT, PET und/oder SPECT umfassen. Insbesondere können zwei oder mehr Modalitäten kombiniert werden, z.B. für eine molekulare Bildgebung.

Die Kombination von zwei oder mehr medizinischen Scannern (und/oder Modalitäten), insbesondere zur molekularen Bildgebung, kann in einem Ausführungsbeispiel PET und CT (kurz: PET/CT) umfassen. In einem weiteren Ausführungsbeispiel kann die Kombination von zwei oder mehr medizinischen Scannern (und/oder Modalitäten), insbesondere zur molekularen Bildgebung, SPECT und CT umfassen (kurz: SPECT/CT).

Die erfindungsgemäße Technik zum Komprimieren eines digitalen medizinischen Bild-Datensatzes kann auf Bild-Daten jeder medizinischen Bildgebungs-Modalität anwendbar sein.

Der digitale medizinische Bild-Datensatz kann zweidimensional (2D) oder dreidimensional (3D, auch: volumetrisch) sein. Beispielsweise kann ein 2D digitaler medizinischer Bild-Datensatz eine Schicht umfassen. Alternativ oder ergänzend kann ein 3D digitaler medizinischer Bild-Datensatz einen Stapel Schichten umfassen.

Der empfangene digitale medizinische Bild-Datensatz (kurz: medizinischer Bild-Datensatz) kann Rohdaten einer Aufnahme des medizinischen Scanners umfassen. Alternativ oder ergänzend kann der medizinische Bild-Datensatz Daten in einem transformierten Raum umfassen. Alternativ oder ergänzend kann der medizinische Bild-Datensatz Daten im k-Raum einer MRT-Aufnahme umfassen. Alternativ oder ergänzend kann der medizinische Bild-Datensatz Daten im (insbesondere komplexen) Bildraum einer Aufnahme des medizinischen Scanners umfassen.

Weiterhin alternativ oder ergänzend kann der medizinische Bild-Datensatz Daten in einem Hybridraum einer MRT-Aufnahme umfassen.

Die Daten im transformierten Raum können mittels einer Fourier-Transformation (FT), Wavelet-Transformation (WT) und/oder Sparsitäts-Transformation (ST) transformiert sein.

Die FT kann beispielsweise eine schnelle FT (fachsprachlich: Fast Fourier Transformation FFT) umfassen.

Die WT (Wavelet Transformation) kann eine lineare Zeit-Frequenz-Transformation umfassen mit einer Wavelet-Analyse, welche den Übergang der Zeitdarstellung in die Spektral- bzw. Waveletdarstellung bezeichnet, und einer Wavelet-Synthese, welche die Rücktransformation der Wavelettransformierten in die Zeitdarstellung bezeichnet. Alternativ oder ergänzend kann die WT eine Weiterbildung der Kurzzeit-FT (fachsprachlich: Short Term Fourier Transformation, STFT) sein.

Mittels einer (z.B. 2D diskreten) Haar-WT kann beispielsweise spektrale Information des medizinischen Bild-Datensatzes (z.B. eines Kopf-Bildes) ausgenutzt werden. Die Haar-WT kann einer diskreten Kosinustransformation entsprechen, welche in einem Bild-Pixel-Rechteck nacheinander in horizontaler und vertikaler Richtung angewandt wird.

Die ST kann eine Sparsität von Daten in einer beliebigen Dimension des Eingangsdatensatzes herstellen. Bekannte ST sind Sobel-, Scharr- oder Laplace-Filter, Singulärwertzerlegung oder Total Variation-Ansätze. Insbesondere kann die ST aber auch aus einer FT oder WT bestehen.

Die Daten im Hybridraum können Fourier-transformiert sein in einer eindimensionalen (1D) Zeitrichtung und/oder Ausleserichtung (auch: Phasenkodierrichtung im k-Raum). Beispielsweise kann eine FT einer Herz-MRT-Zeitserie in Zeitrichtung ausgeführt werden. Dort, wo sich wenig im Bild ändert, finden sich nur Fourier-Koeffizienten mit niedriger Frequenz, der Rest (insbesondere Fourier-Koeffizienten mit hohen Frequenzen) ist annähernd Null.

Die unterschiedlichen Datenarten können verschiedenen Stadien einer Datenverarbeitung des digitalen medizinischen Bild-Datensatzes entsprechen. Alternativ oder ergänzend kann die erfindungsgemäße Technik zum Komprimieren eines digitalen medizinischen Bild-Datensatzes in unterschiedlichen Stadien der Verarbeitung des digitalen medizinischen Bild-Datensatzes angewendet werden.

Die Datenredundanzdimension kann eine Zeitdimension umfassen im Fall, dass der digitale medizinische Bild-Datensatz eine Zeitserienaufnahme enthält. Alternativ oder ergänzend kann die Datenredundanzdimension eine Repetitionsdimension umfassen im Fall, dass der digitale medizinische Bild-Datensatz eine Anzahl Repetitionen umfasst. Alternativ oder ergänzend kann die Datenredundanzdimension eine Kontrastdimension umfassen im Fall, dass der digitale medizinische Bild-Datensatz eine Multi-Kontrastaufnahme enthält. Alternativ oder ergänzend kann die Datenredundanzdimension eine Kanaldimension umfassen im Fall, dass der digitale medizinische Bild-Datensatz eine Mehrkanalaufnahme enthält. Alternativ oder ergänzend kann die Datenredundanzdimension eine Schichtdimension umfassen im Fall, dass der digitale medizinische Bild-Datensatz eine mehrschichtige Aufnahme enthält. Alternativ oder ergänzend kann die Datenredundanzdimension eine Spulendimension umfassen im Fall, dass der digitale medizinische Bild-Datensatz eine Aufnahme mit mehreren Spulen enthält.

Der empfangene Hinweis auf die mindestens einen Datenredundanzdimensionen kann zwei oder mehr Datenredundanzdimensionen umfassen.

Die Datenredundanz in der Zeitdimension kann umfassen, dass einzelne Instanzen (z.B. pro Zeitpunkt der Aufnahme) innerhalb der Zeitserie redundante Informationen umfassen. Beispielsweise können zeitlich benachbarte Instanzen eine große Datenredundanz umfassen. Alternativ oder ergänzend können Instanzen zum selben Zeitpunkt innerhalb eines (z.B. Herz- und/oder Atem-) Zyklus eine große Datenredundanz aufweisen.

Die Datenredundanz in der Repetitionsdimension kann umfassen, dass in dem digitalen medizinische Bild-Datensatz eine Aufnahme mit meiner Vielzahl Repetitionen (z.B. umfassend eine Vielzahl von Atemzyklen und/oder Herzzyklen) enthalten ist.

Die Datenredundanz in der Kontrastdimension kann umfassen, dass redundante Informationen von Aufnahmen mit unterschiedlichen Bildkontrasten in dem digitalen medizinischen Bild-Datensatz miteinander identifizierbar (und/oder kombinierbar) sind. Beispielsweise können T1-gewichtete MRT-Sequenzen (T1w), T2-gewichtete MRT-Sequenzen (T2w), T2*-gewichtete MRT-Sequenzen (T2*w), diffusionsgewichtete MRT-Sequenzen (DWI) und/oder dynamische kontrastmittelangehobene MRT-Sequenzen (DCE) miteinander kombiniert werden. Alternativ oder ergänzend kann eine Dixon-Bildgebung verwendet werden.

Die Dixon-Bildgebung nutzt beispielsweise aus, dass Wasser-Moleküle und Fett-Moleküle mit unterschiedlicher Frequenz prädizieren und mit der Zeit zwischen In-Phase und Gegen-Phase alternieren. Mittels Addition und Subtraktion der In-Phasen- und Gegen-Phasen-Messungen können Wasser-Bilder und Fett-Bilder separiert werden.

Die Datenredundanz in der Kanaldimension kann umfassen, dass redundante Informationen in verschiedenen (z.B. Mess-) Kanälen innerhalb des dem digitalen medizinischen Bild-Datensatz enthalten sind.

Die Datenredundanz in der Schichtdimension kann umfassen, dass (insbesondere benachbarte) Schichten einer im digitalen medizinischen Bild-Datensatz umfassten volumetrischen Aufnahme redundante Informationen enthalten.

Die Ausnutzung der Datenredundanz in der Zeitdimension und/oder in der Schichtdimension kann ein Schiebefenster (fachsprachlich: Sliding Window) für benachbarte Instanzen bzw. Schichten verwenden. Alternativ oder ergänzend können Faltungen auf Nachbarvoxel (und/oder Nachbarpixel) vorgenommen werden, insbesondere für eine Datenredundanz in der Schichtdimension und/oder der Spulendimension.

Die Datenredundanz in der Spulendimension kann umfassen, dass Messungen mehrerer Spulen innerhalb des digitalen medizinischen Bild-Datensatzes redundante Informationen enthalten. Beispielsweise kann die Messung mittels einer Spule einer MRT-Aufnahme einem Kanal entsprechen. Alternativ oder ergänzend kann eine Aufnahme mit mehreren Spulen eine Mehrkanalaufnahme umfassen.

Mittels der erfindungsgemäßen Technik können die redundanten Informationen komprimiert werden. Insbesondere kann der Autoencoder aufgrund des empfangenen Hinweises die Daten gezielt entlang der Datenredundanzdimension komprimieren, z.B. aufgrund eines im Hinweis enthaltenen a-priori-Wissens einer Art der medizinischen Aufnahme.

Der digitale medizinische Bild-Datensatz kann von dem medizinischen Scanner gemäß einem vorbestimmtem Bildgebungsprotokoll erhalten werden.

Das vorbestimmte Bildgebungsprotokoll kann ein Protokoll gemäß dem Human Connectome Project (HCP) umfassen. Alternativ oder ergänzend kann das vorbestimmte Bildgebungsprotokoll ein Protokoll einer funktionellen Kernspintomographie (fMRI, auch: fMRT) umfassen. Alternativ oder ergänzend kann das vorbestimmte Bildgebungsprotokoll ein Protokoll einer Diffusions-Tensor-Bildgebung (fachsprachlich: Diffusion Tensor Imaging, DTI) umfassen. Weiterhin alternativ oder ergänzend kann das vorbestimmte Bildgebungsprotokoll ein Protokoll einer scheinbaren Diffusionskoeffizienten (fachsprachlich: Apparent Diffusion Coefficient, ADC)-Aufnahme umfassen (z.B. eine diffusionsgewichtete, DWI, MRT-Sequenz).

Das HCP kann eine Studienreihe, beispielsweise hinsichtlich des menschlichen Gehirns, umfassen.

Die fMRI kann ein bildgebendes Verfahren umfassen, um physiologische Funktionen im Inneren eines Patientenkörpers mit den Methoden der MRT darzustellen.

Die DTI kann ein bildgebendes Verfahren umfassen, das mit Hilfe der MRT die Diffusionsbewegung von Wassermolekülen in Körpergewebe misst und räumlich aufgelöst darstellt, insbesondere für Untersuchungen des Gehirns.

Bei Kenntnis des vorbestimmten Bildgebungsprotokolls kann der Autoencoder besonders spezifisch für die Komprimierungsaufgabe anhand der Datenredundanzdimension trainiert werden. Alternativ oder ergänzend kann der Autoencoder besonders klein (z.B. bezüglich einer Anzahl Layers und/oder Neuronen) gewählt werden.

Der medizinische Scanner kann einen MRT umfassen. Der mindestens eine komprimierte Teil des digitalen medizinischen Bild-Datensatzes kann ein k-Raum-Zentrum, eine k-Raum-Peripherie und/oder sich abwechselnde unterschiedliche Teile des k-Raums umfassen.

Alternativ oder ergänzend kann der mindestens eine komprimierte Teil des digitalen medizinischen Bild-Datensatzes einen Bereich im Bildraum umfassen, insbesondere ein Zentrum und/oder eine Peripherie des Bildraums.

Beispielsweise können bei einer Herz-Bildgebung (bei der insbesondere Interesse an Aufnahmen des schlagenden Herzens besteht) eine Komprimierung Teile des Bildraums umfassen, um Einfaltungsartefakte zu vermeiden. Insbesondere ist es oft nötig, den Thorax eines Patienten mitaufzunehmen. Z.B. können Bereiche im Bildraum, die Teile des Thorax ohne Herz umfassen, anders (insbesondere stärker) komprimiert werden.

Eine Auswahl des zu komprimierenden Teils des digitalen medizinischen Bild-Datensatzes kann anhand einer medizinischen Fragestellung und/oder eine besonders großen erwarteten Datenredundanz getroffen werden.

Vorteilhafterweise kann nur das (insbesondere k-Raum-) Zentrum des Bild-Datensatzes komprimiert werden, z.B. bei Zeitserien, bei denen vor allem hochfrequente Änderungen zu erwarten sind, z.B. fMRI-Aufnahmen des Kopfes.

Vorteilhafterweise kann nur die (insbesondere k-Raum-) Peripherie des Bild-Datensatzes komprimiert werden, z.B. bei Aufnahmen, bei denen die Struktur gleich bleibt, sich aber der Basis-Kontrast ändert, z.B. bei Multi-Kontrast-Aufnahmen und/oder in der Dixon-Bildgebung.

Vorteilhafterweise können abwechselnd unterschiedliche Teile des Bild-Datensatzes (insbesondere des k-Raums) komprimiert werden, z.B. bei Daten, die später noch in einem weiteren Rekonstruktionsschritt weiterverarbeitet werden und bei denen eine verlustbehaftete Komprimierung (auch: Kompression) an einzelnen Zeitpunkten gut toleriert werden kann, solange sie nicht immer die gleichen räumlichen Anteile des Bildes betrifft, z.B. bei radialer (und/oder spiraler) oder Magnetic Resonance Fingerprinting (MRF)-Bildgebung.

Konventionellerweise kann die MRT-Bildgebung eine kartesische Abtastung umfassen, z.B. Zeile für Zeile in einer k-Raum-Matrix. Alternativ oder ergänzend können k-Raum-Zeilen nichtkartesisch aufgenommen werden, z.B. radial oder spiral, wie von Pedro F. Ferreira et al. in "Cardiovascular magnetic resonance artefacts", J Cardiovasc Magn Reson 15, 41 (2013) beschrieben, dessen Inhalt hierin durch Bezugnahme aufgenommen ist. Bei der CT-Bildgebung kann ein analoges Prinzip angewendet werden, indem Röntgenquelle und Detektor (z.B. kreisförmig) um den Patienten gedreht werden.

Die MRF-Bildgebung ist z.B. eine Art von quantitiver MRT-Bildgebung, bei der eine pseudowillkürliche Aufnahmestrategie angewendet wird. Eindeutige Signalmuster (auch; "Fingerprints") werden erzeugt für unterschiedliche Materialien und/oder Gewebearten, wonach ein Mustererkennungsalgorithmus die "Fingerprints" auf ein vorbestimmtes Lexikon erwarteter Signalmuster abbildet.

Durch das nur teilweise Komprimieren kann die erfindungsgemäße Technik besonders schnell und ressourcenschonend ausgeführt werden.

Das Komprimieren des mindestens einen Teils des empfangenen digitalen medizinischen Bild-Datensatzes kann ferner basieren auf (und/oder der empfangene Hinweis auf die mindestens eine Datenredundanzdimension kann umfassen) eine(r) Sliding-Window-Kodierung benachbarter Schichten innerhalb des Bild-Datensatzes und/oder benachbarter zeitlicher Instanzen einer Zeitserienaufnahme innerhalb des Bild-Datensatzes. Das Komprimieren basierend auf der Sliding-Window-Kodierung kann insbesondere im Sinne eines Komprimierens anhand der Datenredundanzdimension und/oder zusätzlich zum Komprimieren anhand einer weiteren Datenredundanzdimension erfolgen.

Bei der Sliding-Window-Kodierung können eine Vielzahl (z.B. 20) Schichten einzeln kodiert werden. Als Eingangsdaten (kurz: Eingang) des Autoencoders können neben der zu kodierenden Schicht auch die (z.B. beiden) Nachbarschichten (bzw. bei Randschichten z.B. eine Matrix mit Nullen) kodiert werden, um die Komprimierung zu verbessern.

Die Sliding-Window-Kodierung kann äquivalent sein zum lediglichen Speichern von Unterschieden einer Schicht relativ zu einer (z.B. ersten) Referenzschicht.

Ein weiterer Aspekt der Sliding Windows-Kodierung kann umfassen, dass der Autoencoder für eine feste Anzahl an Schichten lernt (bzw. trainiert wird), aber auf eine beliebige Anzahl an Schichten anwendbar ist. Werden z.B. fünf (5) Schichten kodiert und wird mit Schrittweite 1 über die 20 Eingangsschichten gefahren, so erhöht dies zunächst die Anzahl der zu übertragenden Datenpakete. Dem kann vorgebeugt werden, indem die Schrittweite größer gewählt wird (auch als "Jumping Window" bezeichenbar). Alternativ oder ergänzend kann die Daten-Komprimierung durch die zwischen den Schichten bestehende Redundanz erhöht und somit die erhöhte Anzahl an zu übertragenden Datenpaketen ausglichen werden. Alternativ oder ergänzend über Schichten, die in mehr als einem Datenpaket übertragen wurden (z.B. kann Schicht 3 bei drei Schichten in der Extradimension an Position 5, 4, 3, 2, 1 in den jeweiligen Datenpaketen 1, 2, 3, 4 und 5 übertragen werden), nach dem Empfang gemittelt werden.

Alternativ oder ergänzend kann das Komprimieren des mindestens einen Teils des empfangenen digitalen medizinischen Bild-Datensatzes ferner basieren auf (und/oder der empfangene Hinweis auf die mindestens eine Datenredundanzdimension kann umfassen) eine(r) Symmetrieeigenschaft des Bild-Datensatzes, insbesondere einer hermiteschen k-Raum-Symmetrie einer MRT-Aufnahme. Das Komprimieren basierend auf der hermiteschen k-Raum-Symmetrie kann umfassen, dass die vier Quadranten des k-Raums redundante Informationen enthalten, die durch Spiegelungen und komplexe Konjugation aufeinander abgebildet werden können.

Das Komprimieren basierend auf der Symmetrieeigenschaft des Bild-Datensatzes kann insbesondere im Sinne eines Komprimierens anhand der Datenredundanzdimension und/oder zusätzlich zum Komprimieren anhand einer weiteren Datenredundanzdimension erfolgen. Beispielsweise kann der k-Raum gegenüber seinem Zentrum (z.B ideal) als annährend (und/oder idealerweise) punktsymmetrisch angesehen werden. Wird der k-Raum in vier Quadranten unterteilt, die so übereinander klappbar sind, dass das Zentrum jeweils an der gleichen Ecke angeordnet ist, so wird dem Autencoder ermöglicht, räumliche Korrelationen und Redundanzen zu lernen, die sich aus einer (insbesondere auch real existierenden) gewissen Ähnlichkeit der Quadranten ergeben können.

Weiterhin alternativ oder ergänzend kann das Komprimieren des mindestens einen Teils des empfangenen digitalen medizinischen Bild-Datensatzes ferner basieren auf einer Überabtastung entlang einer Ausleserichtung, insbesondere für einen Bild-Datensatz umfassend Daten im Hybridraum einer MRT-Aufnahme. Das Komprimieren basierend auf der Überabtastung kann insbesondere zusätzlich zum Komprimieren anhand der Datenredundanzdimension erfolgen. Die Überabtastung (fachsprachlich: Oversampling) kann umfassen, dass mehr Datenmessungen eines MRT-Signals durchgeführt werden, als für die Auflösung der Bildanzeige erforderlich sind. Z.B. kann das MRT-Signal 512 Mal bis 1024 Mal pro Echo abgetastet werden (insbesondere auch wenn die Anzeigeauflösung in der Frequenzcodierrichtung in der Regel mit 256 angegeben wird). Alternativ oder ergänzend kann eine Nyquist-Abtastrate (auch: Nyquist-Frequenz) mindestens das zweifache (z.B. bis vierfache) der höchsten Frequenz des Signals sein. Da diese Aufgabe lediglich durch eine Erhöhung der Digitalisierungsrate eines Abtastschaltkreises erfüllt wird, verursacht sie im Wesentlichen keinen Zeitverlust und erfolgt "unsichtbar".

Die Abtastung mit der Nyquist-Frequenz kann in Ausleserichtung erfolgen.

Im Fall, dass keine Übertabtastung mit der Nyquist-Frequenz erfolgt, kann eine Fehlausrichtung (fachsprachlich: Misalignment) bei hoher Frequenz stattfinden, wenn niedrigere Frequenzen auftreten.

Durch geeignetes Überabtasten können Bereiche, in denen fast kein Signal auftritt, gut komprimierbar sein.

Der Überabtastungsfaktor kann einstellbar sein kann.

Der Autoencoder kann auf die Datenredundanzdimension trainiert werden. Das Training kann einen Schritt des Empfangens eines Hinweises auf mindestens eine Datenredundanzdimension eines digitalen medizinischen Trainings-Bild-Datensatzes (kurz: Trainings-Bild-Datensatz) an (insbesondere der zumindest einen Extradimension) der Eingangsschicht eines Autoencoders umfassen. Der Trainings-Bild-Datensatz kann mittels eines medizinischen Scanners erhalten worden sein. Alternativ oder ergänzend kann der Trainings-Bild-Datensatz synthetisch erzeugt worden sein.

Das Training des Autoencoders kann ferner einen Schritt des Empfangens des digitalen medizinischen Trainings-Bild-Datensatzes an der Eingangsschicht des Autoencoders umfassen. Das Empfangen des digitalen medizinischen Trainings-Bild-Datensatzes kann insbesondere ein Empfangen von Daten im Hinblick auf die Datenredundanzdimension an der zumindest einen Extradimension der Eingangsschicht umfassen. Vorzugsweise können die an der zumindest einen Extradimension in der Eingangsschicht empfangenen Daten zumindest einen, insbesondere im Hinblick auf die Datenredundanzdimension ausgewählten, Teil des empfangenen digitalen medizinischen Trainings-Bild-Datensatzes umfassen.

Ferner kann das Training einen Schritt des Komprimierens, mittels verborgener Schichten des Autoencoders, mindestens eines Teils des empfangenen digitalen medizinischen Test-Bild-Datensatzes umfassen. Insbesondere kann das Komprimieren unter Verwendung des empfangenen Hinweises auf die mindestens eine Datenredundanzdimension des digitalen medizinischen Trainings-Bild-Datensatzes und/oder unter Verwendung von an der zumindest einen Extradimension empfangenen Daten erfolgen.

Das Training kann weiterhin einen Schritt des Ausgebens (und/oder Übertragen) des Ergebnisses des Komprimierens des mindestens einen Teils des empfangenen digitalen medizinischen Test-Bild-Datensatzes von einer Ausgangsschicht des Autoencoders an eine Eingangsschicht eines Autodecoders umfassen. Das Ausgeben (und/oder Übertragen) des Ergebnisses kann einen an der Ausgabeschicht des Autoencoders bereitgestellten Hinweis auf die beim Komprimieren verwendete Datenredundanzdimension umfassen, der an die Eingabeschicht des Autodecoders übertragen wird, beispielsweise konkateniert mit den komprimierten digitalen medizinischen Test-Bild-Datensatz.

Das Training kann ferner einen Schritt des Dekomprimierens, mittels verborgener Schichten des Autodecoders, des Ergebnisses des Komprimierens des mindestens einen Teils des empfangenen digitalen medizinischen Test-Bild-Datensatzes umfassen, insbesondere basierend auf dem übertragenen Hinweis auf die beim Komprimieren verwendete Datenredundanzdimension. Das Training kann ferner einen Schritt des Ausgebens, insbesondere an einer Ausgangsschicht des Autodecoders, des Ergebnisses des Dekomprimierens umfassen sowie ein Anwenden einer Verlustfunktion auf den empfangenen digitalen medizinischen Trainings-Bild-Datensatz und auf das ausgegebene Ergebnis des Dekomprimierens.

Zumindest die Trainings-Schritte des Komprimierens durch den Autoencoder, des Ausgebens vom Autoencoder zum Autodecoder und des Dekomprimierens durch den Autodecoder können wiederholt werden, um die angewendete Verlustfunktion zu optimieren.

Das Trainieren des Autoencoders kann ein unüberwachtes Lernen umfassen.

Der Autoencoder und der Autodecoder können gemeinsam trainiert werden. Beispielsweise können beim Optimieren der angewendeten Verlustfunktion Gewichte von Neuronen des Autoencoders und/oder Gewichte von Neuronen des Autodecoders modifiziert werden.

Zumindest in einer auf die Trainingsphase folgenden Inferenzphase kann der trainierte Autodecoder räumlich getrennt sein vom trainierten Autoencoder. Beispielsweise kann der trainierte Autoencoder in räumlicher Nähe zu einem oder mehreren medizinischen Scannern (z.B. in einer Radiologieabteilung) angeordnet sein. Der trainierte Autodecoder (und/oder eine Anzahl von Kopien des trainierten Autodecoders) kann in einer (insbesondere medizinischen) Einrichtung angeordnet sein, die zum Erhalt von medizinischen Patientendaten berechtigt ist. Beispielsweise kann eine Kopie des Autodecoders in einer Praxis eines überweisenden Arztes lokalisiert sein.

Der digitale medizinische Trainings-Bild-Datensatz kann beispielsweise einen HCP-Datensatz (insbesondere einen HCP-Diffusionsdatensatz) und/oder einen fMRI-Datensatz umfassen.

Die Verlustfunktion kann eine MSE, eine MAE und/oder einen SSIM umfassen.

Das Training kann ferner eine Rückpropagation und/oder ein Gradientenverfahren zum Trainieren des Autoencoders umfassen.

Mittels der Rückpropagation (fachsprachlich: Backpropagation) und/oder des Gradientenverfahrens kann eine Konvergenz des Trainings verbessert werden.

Der Autoencoder kann groß dimensioniert sein und/oder eine Möglichkeit zum Auffüllen mit Nullen von Eingangsdaten aufweisen, insbesondere abhängig von einer Art von Datenredundanzdimension und digitalem medizinischen Trainings-Bild-Datensatz. Der Autoencoder kann mit einer Mehrzahl unterschiedlicher Arten von digitalen medizinischen Trainings-Bild-Datensätzen und Datenredundanzdimensionen trainiert werden.

Ein Autoencoder, der für eine fest vorbestimmte Art von Anwendungsfall trainiert ist (z.B. Datenredundanzreduktion in einer Spulendimension einer MRT-Aufnahme eines Kniegelenks), kann klein dimensioniert sein.

Klein dimensioniert und groß dimensioniert kann sich auf eine Anzahl Neuronen pro Schicht (z.B. Eingangsschicht, verborgene Schicht und/oder Ausgangsschicht) beziehen und/oder auf eine Anzahl (insbesondere verborgener) Schichten.

Ein Autoencoder, der für eine Mehrzahl von Anwendungsfällen trainiert ist (z.B. Datenredundanzreduktion in Zeit-, Schicht- und/oder Spulendimension von MRT-Aufnahmen unterschiedlicher Körperteile), kann groß dimensioniert sein.

Das Auffüllen mit Nullen (fachsprachlich: Zero-Filling) von Eingangsdaten kann abhängig vom Anwendungsfall sein. Beispielsweise kann für einen vorbestimmten Anwendungsfall eine vorbestimmte Anzahl Neuronen der Eingangsschicht nicht aktiviert werden.

Das Auffüllen mit Nullen kann z.B. für 256 Datenpunkte ein Einfügen von links und rechts je 128 Nullen umfassen, um eine vorbestimmte (und/oder gewünschte) Datensatz-Größe von 512 zu erreichen.

Gemäß einem Vorrichtungsaspekt wird ein trainiertes Autoencoder-Netzwerk (kurz: Autoencoder) zum Komprimieren eines digitalen medizinischen Bild-Datensatzes, der mittels eines medizinischen Scanners erhalten wurde, bereitgestellt. Eine Eingangsschicht des trainierten Autoencoder-Netzwerks ist zum Empfangen des digitalen medizinischen Bild-Datensatzes ausgebildet. Die Eingangsschicht des trainierten Autoencoder-Netzwerks kann ferner zum Empfangen eines Hinweises auf mindestens eine Datenredundanzdimension des empfangenen digitalen medizinischen Bild-Datensatzes ausgebildet sein. Die Eingangsschicht des trainierten Autoencoder-Netzwerks kann zumindest eine Extradimension in der Eingangsschicht umfassen. Die zumindest eine Extradimension kann dazu ausgebildet sein, den Hinweis auf mindestens eine Datenredundanzdimension des empfangenen digitalen medizinischen Bild-Datensatzes zu empfangen. Alternativ oder ergänzend kann die zumindest eine Extradimension in Antwort auf einen empfangenen Hinweis auf mindestens eine Datenredundanzdimension des empfangenen digitalen medizinischen Bild-Datensatzes bereitgestellt werden. Alternativ oder ergänzend kann die zumindest eine Extradimension in der Eingangsschicht zum Empfangen von Daten im Hinblick auf die Datenredundanzdimension ausgebildet sein. Vorzugsweise können die an der zumindest einen Extradimension in der Eingangsschicht empfangenen Daten zumindest einen (insbesondere im Hinblick auf die Datenredundanzdimension ausgewählten) Teil des empfangenen digitalen medizinischen Bild-Datensatzes umfassen.

Das trainierte Autoencoder-Netzwerk ist zum Komprimieren mindestens eines Teils des empfangenen digitalen medizinischen Bild-Datensatzes unter Verwendung des Hinweises auf die mindestens eine Datenredundanzdimension und/oder unter Verwendung von an der zumindest einen Extradimension empfangenen Daten ausgebildet.

Eine Ausgangsschicht des trainierten Autoencoder-Netzwerks kann optional zumindest eine (z.B. in Antwort auf einen empfangenen Hinweis auf mindestens eine Datenredundanzdimension des empfangenen digitalen medizinischen Bild-Datensatzes bereitgestellte) Extradimension umfassen. Die in der Ausgangsschicht bereitgestellte zumindest eine Extradimension kann vorzugsweise dazu ausgebildet sein, einen Hinweis auf die zum Komprimieren verwendete Datenredundanzdimension zu umfassen und/oder bereitstellen.

Das Autoencoder-Netzwerk kann zum Ausführen des Verfahrens gemäß dem Verfahrensaspekt ausgebildet sein. Alternativ oder ergänzend kann das Autoencoder-Netzwerk gemäß dem Verfahrensaspekt trainiert sein. Weiterhin alternativ oder ergänzend kann das Autoencoder-Netzwerk eines oder jedes Merkmal umfassen, dass im Kontext des Verfahrensaspekts für den Autoencoder offenbart ist.

Gemäß einem Systemaspekt wird ein System für zum Komprimieren eines digitalen medizinischen Bild-Datensatzes, der mittels eines medizinischen Scanners erhalten wurde, bereitgestellt. Das System umfasst eine Scannerschnittstelle zu mindestens einem medizinischen Scanner und zumindest ein Autoencoder-Netzwerk gemäß dem Vorrichtungsaspekt.

Das System kann eine Mehrzahl Autoencoder-Netzwerke umfassen. Beispielsweise kann je ein Autoencoder-Netzwerk für eine Art Anwendungsfall trainiert sein.

Gemäß einem weiteren Aspekt wird ein Computerprogrammprodukt bereitgestellt mit Programmelementen, die einen Autoencoder-Netzwerk veranlassen, die Schritte des Verfahrens zum Komprimieren eines digitalen medizinischen Bild-Datensatzes, der mittels eines medizinischen Scanners erhalten wurde, und/oder zum Trainieren des Autoencoder-Netzwerks gemäß dem Verfahrensaspekt auszuführen, wenn die Programmelemente in einen Speicher des Autoencoder-Netzwerk geladen werden.

Gemäß einem noch weiteren Aspekt wird ein computerlesbares Medium bereitgestellt, auf dem Programmelemente gespeichert sind, die von einem Autoencoder-Netzwerk gelesen und ausgeführt werden können, um Schritte des Verfahrens zum Komprimieren eines digitalen medizinischen Bild-Datensatzes, der mittels eines medizinischen Scanners erhalten wurde, und/oder zum Trainieren des Autoencoder-Netzwerks gemäß dem Verfahrensaspekt durchzuführen, wenn die Programmelemente von dem Autoencoder-Netzwerk ausgeführt werden.

Die oben beschriebenen Eigenschaften, Merkmale und Vorteile der vorliegenden Erfindung sowie die Art und Weise, wie sie erreicht werden, werden im Lichte der folgenden Beschreibung und der Ausführungsbeispiele, die im Zusammenhang mit den Zeichnungen näher erläutert werden, klarer und verständlicher. Diese folgende Beschreibung beschränkt die Erfindung nicht auf die enthaltenen Ausführungsformen. Gleiche Komponenten oder Teile können in verschiedenen

Figuren mit den gleichen Bezugszeichen versehen sein. Im Allgemeinen sind die Abbildungen nicht maßstabsgetreu.

Es versteht sich, dass eine bevorzugte Ausführungsform der vorliegenden Erfindung auch eine beliebige Kombination der abhängigen Ansprüche oder der obigen Ausführungsformen mit dem jeweiligen unabhängigen Anspruch sein kann.

Diese und andere Aspekte der Erfindung werden aus den nachfolgend beschriebenen Ausführungsformen ersichtlich und werden durch Bezugnahme auf diese erläutert.

### KURZE BESCHREIBUNG DER ZEICHNUNGEN

Fig. 1 ist ein Flussdiagramm eines Verfahrens zum Komprimieren eines digitalen medizinischen Bild-Datensatzes, der mittels eines medizinischen Scanners erhalten wurde, gemäß einer bevorzugten Ausführungsform der vorliegenden Erfindung;
Fig. 2 ist ein Flussdiagramm einer Trainingsphase eines Autoencoders, um später in einer Inferenzphase das Verfahren zum Komprimieren eines digitalen medizinischen Bild-Datensatzes, der mittels eines medizinischen Scanners erhalten wurde, gemäß Fig. 1 auszuführen;
Fig. 3 ist eine Übersicht über die Struktur und den Aufbau eines Autoencoder-Netzwerks gemäß einer bevorzugten Ausführungsform der vorliegenden Erfindung, wobei das Autoencoder-Netzwerk gemäß der Trainingsphase der Fig. 2 zum Ausführen des Verfahrens der Fig. 1 ausgebildet ist;
Fig. 4 zeigt ein herkömmliches faltendes Autoencoderdecoder-Netzwerk zur herkömmlichen 2D-Bildkompression; und
Fig. 5 zeigt beispielhaft eine Kopf-Aufnahme mit Haar-Wavelet-Transformationen.

Etwaige Bezugszeichen in den Ansprüchen sind nicht als Einschränkung des Anwendungsbereichs zu verstehen.

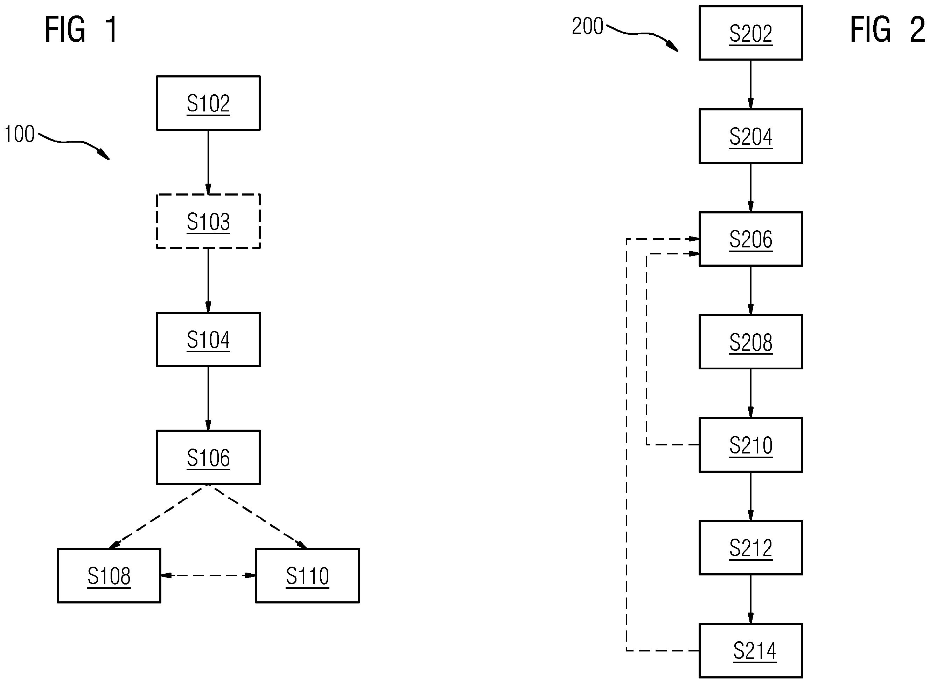

Fig. 1 zeigt schematisch ein beispielhaftes Ablaufdiagramm eines (insbesondere computerimplementierten) Verfahrens 100 zum Komprimieren eines digitalen medizinischen Bild-Datensatzes, der mittels eines medizinischen Scanners erhalten wurde. Das Verfahren 100 kann in einer Inferenzphase eines trainierten Autoencoders ausgeführt werden.

Das Verfahren 100 umfasst einen Schritt S102 des Empfangens eines Hinweises auf mindestens eine Datenredundanzdimension eines digitalen medizinischen Bild-Datensatzes.

In Antwort auf den empfangenen Hinweis auf die mindestens eine Datenredundanzdimension kann ein trainierter Autoencoder ausgewählt werden (nicht gezeigt).

Das Verfahren 100 umfasst ferner einen Schritt S104 des Empfangens des digitalen medizinischen Bild-Datensatzes von einem medizinischen Scanner an einer Eingangsschicht des trainierten Autoencoders.

Der trainierte Autoencoder kann zumindest eine Extradimension in der Eingangsschicht umfassen. Optional umfasst der trainierte Autoencoder ferner zumindest eine Extradimension in einer Ausgangsschicht.

Die zumindest eine Extradimension (insbesondere in der Eingangsschicht) kann dazu ausgebildet sein, den Hinweis auf mindestens eine Datenredundanzdimension des empfangenen digitalen medizinischen Bild-Datensatzes zu empfangen S102. Alternativ oder ergänzend wird die zumindest eine Extradimension (insbesondere in der Eingangsschicht, und optional in der Ausgangsschicht) in Antwort auf den empfangenen S102 Hinweis auf die mindestens eine Datenredundanzdimension des empfangenen digitalen medizinischen Bild-Datensatzes bereitgestellt. Weiterhin alternativ oder ergänzend ist die zumindest eine Extradimension in der Eingangsschicht zum Empfangen von Daten im Hinblick auf die Datenredundanzdimension ausgebildet. Vorzugsweise umfassen die an der zumindest einen Extradimension in der Eingangsschicht empfangenen Daten zumindest einen (insbesondere im Hinblick auf die Datenredundanzdimension ausgewählten) Teil des empfangenen S104 digitalen medizinischen Bild-Datensatzes.

Das Verfahren 100 umfasst ferner einen Schritt S106 des Komprimierens mindestens eines Teils des empfangenen S104 digitalen medizinischen Bild-Datensatzes unter Verwendung des empfangenen S102 Hinweises auf die mindestens eine Datenredundanzdimension mittels des trainierten Autoencoders und/oder unter Verwendung von an der zumindest einen Extradimension empfangenen Daten.

Optional kann das Verfahren 100 einen Schritt S108 des Übertragens des komprimierten S106 digitalen medizinischen Bild-Datensatzes umfassen. Alternativ oder ergänzend kann das Verfahren 100 einen Schritt S110 des Speicherns des komprimierten S106 digitalen medizinischen Bild-Datensatzes umfassen.

Weiterhin optional kann das Verfahren 100 einen Schritt S103 des Bestimmens eines Kompressionsfaktors für das Komprimieren S106 des mindestens einen Teils des empfangenen S104 digitalen medizinischen Bild-Datensatzes umfassen.

Fig. 2 zeigt schematisch ein beispielhaftes Ablaufdiagramm einer Trainingsphase 200 eines Autoencoders auf die Datenredundanzdimension. Der Autoencoder kann nach der Trainingsphase 200 in der Inferenzphase das Verfahren 100 zum Komprimieren eines digitalen medizinischen Bild-Datensatzes ausführen.

Die Trainingsphase 200 kann einen Schritt S202 des Empfangens eines Hinweises auf mindestens eine Datenredundanzdimension eines digitalen medizinischen Trainings-Bild-Datensatzes an der Eingangsschicht eines Autoencoders, insbesondere an zumindest einer Extradimension der Eingangsschicht, umfassen.

Der digitale medizinische Trainings-Bild-Datensatz kann mittels eines medizinischen Scanners erhalten worden sein. Alternativ oder ergänzend kann der digitale medizinische Trainings-Bild-Datensatz synthetisch erzeugt worden sein, beispielsweise mittels eine Generative Adversarial Networks (GAN), mittels eines Variational Autoencoders (VAE) und/oder mittels eines anderen geeigneten Neuronalen Netzwerkes.

Die Trainingsphase 200 kann ferner einen Schritt S204 des Empfangens des digitalen medizinischen Trainings-Bild-Datensatzes an der Eingangsschicht des Autoencoders umfassen. Insbesondere der Schritt S204 ein Empfangen von Daten im Hinblick auf die Datenredundanzdimension an der zumindest einen Extradimension der Eingangsschicht umfassen. Vorzugsweise können die an der zumindest einen Extradimension in der Eingangsschicht empfangenen Daten zumindest einen (insbesondere im Hinblick auf die Datenredundanzdimension ausgewählten) Teil des empfangenen S204 digitalen medizinischen Trainings-Bild-Datensatzes umfassen.

Die Trainingsphase 200 kann ferner einen Schritt S206 des Komprimierens mindestens eines Teils des empfangenen S204 digitalen medizinischen Test-Bild-Datensatzes mittels verborgener Schichten des Autoencoders umfassen. Insbesondere kann das Komprimieren S206 unter Verwendung des empfangenen S202 Hinweises auf die mindestens eine Datenredundanzdimension des digitalen medizinischen Trainings-Bild-Datensatzes und/oder unter Verwendung von an der zumindest einen Extradimension empfangenen Daten erfolgen.

Die Trainingsphase 200 kann ferner einen Schritt S208 des Ausgebens des Ergebnisses des Komprimierens S208 des mindestens einen Teils des empfangenen S204 digitalen medizinischen Test-Bild-Datensatzes von einer Ausgangsschicht des Autoencoders an eine Eingangsschicht eines Autodecoders umfassen. Das Ausgeben S208 des Ergebnisses kann einen an der Ausgabeschicht des Autoencoders bereitgestellten Hinweis auf die beim Komprimieren S206 verwendete Datenredundanzdimension umfassen, der an die Eingabeschicht des Autodecoders übertragen wird, beispielsweise konkateniert mit den komprimierten digitalen medizinischen Test-Bild-Datensatz.

Die Trainingsphase 200 kann ferner einen Schritt S210 des Dekomprimierens des Ergebnisses des Komprimierens S208 des mindestens einen Teils des empfangenen S204 digitalen medizinischen Test-Bild-Datensatzes mittels verborgener Schichten des Autodecoders umfassen, insbesondere basierend auf dem übertragenen S208 Hinweis auf die beim Komprimieren S206 verwendete Datenredundanzdimension.

Die Trainingsphase 200 kann ferner die Schritte S212 und S214 des Ausgebens des Ergebnisses des Dekomprimierens S210 bzw. des Anwenden einer Verlustfunktion auf den empfangenen S204 digitalen medizinischen Trainings-Bild-Datensatz und das ausgegebenen S212 Ergebnis des Dekomprimierens S210 umfassen.

Zumindest die Schritte des Komprimierens S206 durch den Autoencoder, des Ausgebens S208 vom Autoencoder zum Autodecoder und des Dekomprimierens S210 durch den Autodecoder können wiederholt werden, um die angewendete S214 Verlustfunktion zu optimieren.

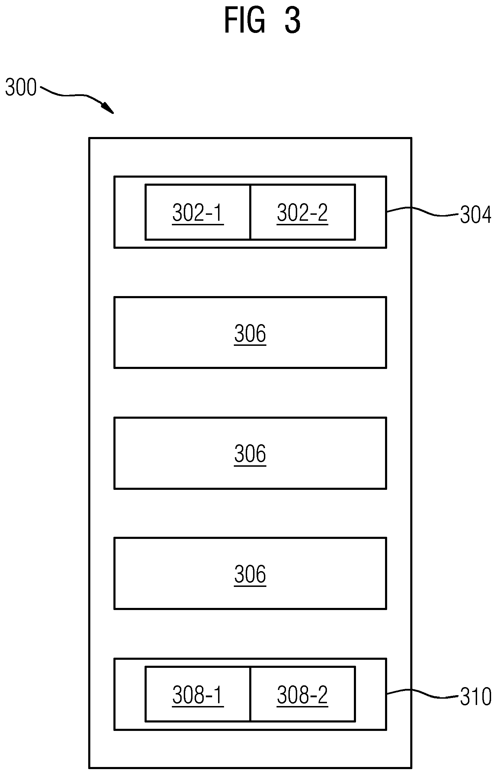

Fig. 3 zeigt schematisch eine Ausführungsbeispiel eines (insbesondere trainierten) Autoencoder-Netzwerks 300 zum Komprimieren eines digitalen medizinischen Bild-Datensatzes, der mittels eines medizinischen Scanners erhalten wurde.

Das Autoencoder-Netzwerk 300 umfasst eine Eingangsschicht 304. Die Eingangsschicht 304 umfasst einen Anteil 302-1, der zum konventionellen Empfangen des digitalen medizinischen Bild-Datensatzes ausgebildet ist. Die Eingangsschicht 304 umfasst optional ferner zumindest eine Extradimension 302-2. Die zumindest eine Extradimension 302-2 kann dazu ausgebildet sein, einen Hinweis auf mindestens eine Datenredundanzdimension des empfangenen digitalen medizinischen Bild-Datensatzes zu empfangen. Alternativ oder ergänzend kann die zumindest eine Extradimension 302-2 in Antwort auf einen empfangenen Hinweis auf mindestens eine Datenredundanzdimension des empfangenen digitalen medizinischen Bild-Datensatzes bereitgestellt werden. Weiterhin alternativ oder ergänzend kann die zumindest eine Extradimension 302-2 in der Eingangsschicht 304 zum Empfangen von Daten im Hinblick auf die Datenredundanzdimension ausgebildet sein. Vorzugsweise können die an der zumindest einen Extradimension 302-2 in der Eingangsschicht 304 empfangenen Daten zumindest einen (insbesondere im Hinblick auf die Datenredundanzdimension ausgewählten) Teil des empfangenen digitalen medizinischen Bild-Datensatzes umfassen.

Das Autoencoder-Netzwerk 300 umfasst eine Mehrzahl verborgener Schichten 306. Die verborgenen Schichten 306 können insbesondere faltende Schichten, ReLU-Schichten und Max Pooling-Schichten umfassen (nicht im Detail gezeigt).

Das Autoencoder-Netzwerk 300 umfasst ferner eine Ausgangsschicht 310. Die Ausgangsschicht 310 umfasst einen Anteil 308-1, der zum konventionellen Ausgeben des komprimierten digitalen medizinischen Bild-Datensatzes ausgebildet ist. Die Ausgangsschicht 310 im Ausführungsbeispiel der Fig. 3 kann ferner zumindest eine Extradimension 308-2 umfassen. Die zumindest eine Extradimension 308-2 kann in Antwort auf einen (insbesondere an der Eingangsschicht 304, speziell an der zumindest einen Extradimension 302-2) empfangenen Hinweis auf mindestens eine Datenredundanzdimension des empfangenen digitalen medizinischen Bild-Datensatzes bereitgestellt werden. Die Extradimension 308-2 kann vorzugsweise einen Hinweis hinsichtlich der verwendeten mindestens einen Datenredundanzdimension zum späteren Dekodieren durch einen (z.B. räumlich getrennten) Decoder bereitstellen, beispielsweise mittels Konkatenieren des Hinweises mit dem komprimierten digitalen medizinischen Bild-Datensatz.

Das trainierte Autoencoder-Netzwerk 300 ist zum Komprimieren mindestens eines Teils des empfangenen digitalen medizinischen Bild-Datensatzes unter Verwendung eines Hinweises auf die mindestens eine Datenredundanzdimension ausgebildet.

Das Autoencoder-Netzwerk 300 kann zum Ausführen des Verfahrens 100 in einer Inferenzphase ausgebildet sein. Alternativ oder ergänzend kann das Autoencoder-Netzwerk mittels der Trainingsphase 200 trainiert sein.

Ein System (nicht gezeigt) zum Komprimieren eines digitalen medizinischen Bild-Datensatzes, der mittels eines medizinischen Scanners erhalten wurde, kann eine Scannerschnittstelle zu mindestens einem medizinischen Scanner und zumindest ein Autoencoder-Netzwerk 300 umfassen. Insbesondere kann das System eine Mehrzahl medizinischer Scanner (z.B. mit unterschiedlichen Modalitäten) und/oder eine Mehrzahl Autoencoder-Netzwerke 300 (z.B. jeweils trainiert auf einen vorbestimmten Anwendungsfall) umfassen.

Die erfindungsgemäße Technik (insbesondere umfassend das Verfahren 100, die Trainingsphase 200, das Autoencoder-Netzwerk 300 und/oder das System) kann insbesondere eine Methode zur Kompression von MRT-Daten mittels Autoencoder-Netzen sein.

Mittels der erfindungsgemäßen Technik wird das Prinzip des Autoencoders und verwandter Netzarchitekturen auf die medizinische (z.B. MRT-) Bildgebung übertragen. Mögliche Anwendungen liegen in der kompakten Speicherung großen Datensätze (z.B. Human Connectome Project (HCP)-Diffusions- und fMRI-Datensätze) und/oder in der schnellen Übertragung von Rohdaten über ein Netzwerk bzw. das Internet. Letzteres ist z.B. für Anwendungsfälle interessant, bei denen Rekonstruktionsaufgaben in eine Cloud oder auf Rechencluster ausgelagert werden sollen.

Die Grundidee der erfindungsgemäßen Technik ist die Datenredundanz in der medizinischen (z.B. MRT-) Bildgebung. Die Datenredundanz wird u.a. genutzt, indem die Dimension, in der eine Redundanz erwartet wird, als zumindest eine Extradimension in der Eingangsschicht (und optional in der Ausgangsschicht) verwendet wird. In der konventionellen nicht-medizinischen 2D-Bildkompression würde diese Extradimension von den RGB-Kanälen aufgespannt werden.

Für medizinische Zeitserienaufnahmen, wie fMRI oder Kontrastmittel-Bildgebung, bildet beispielsweise die Zeitdimension die Extradimension.

Bei konventioneller medizinischer 2D-Bildgebung kann die Extradimension die Kanaldimension oder die Schichtdimension umfassen. Je nach erwarteter Redundanz kann es sinnvoll sein, als Eingangsdaten den k-Raum, den Bildraum oder einen Hybridraum zu verwenden. Außer 2D-Bildgebung sind auch andere Trajektorien denkbar. Für Spiral- oder Radial-Bildgebung kann beispielsweise die zeitliche Abfolge einer ADC-Aufnahme, z.B. auch eine räumliche Dimension der Eingangsschicht des Autoencoders, die Spulendimension in der anderen räumlichen Dimension und die Zeitdimension als Extradimension angeordnet (und/oder sortiert) werden.

Alternativ oder ergänzend kann es einen Zusammenhang sowohl zum Aufnahmezeitpunkt aus auch zur (z.B. tatsächlichen) k-Raum-Position geben. Z.B. kann statt, wie üblich k-Raum-Zeile neben k-Raum Zeile nebeneinander anzuordnen, vorzugsweise die zeitliche Nachbarschaft in der Extradimension kodiert werden. Beispielsweise kann die Spulendimension als zweite räumliche Dimension verwendet werden.

Für vorbestimmte (und/oder spezifische) Bildgebungsprotokolle, die immer mit den gleichen Bildgebungsparametern ausgeführt werden (z.B. HCP-DTI-Protokolle oder fMRI-Protokolle) können dedizierte Autoencoder trainiert werden. Durch die spezifische Abstimmung auf diese Daten sind besonders gute Kompressionsergebnisse erhaltbar.

Für klinische 2D/3D-Bildgebung, bei der die Matrixgröße, Schichtanzahl und Spulenanzahl oft variiert, können unterschiedliche Ansätze (auch kombinierbar) gewählt werden. In einem ersten Ausführungsbeispiel erfolgt ein Training mehrerer Autoencoder-Netze auf unterschiedliche Dimensionen bzw. Anwendungsfälle.

In einem zweiten (mit dem ersten Ausführungsbeispiel kombinierbaren) Ausführungsbeispiel erfolgt ein Training eines oder mehrerer Autoencoder-Netze mit hohen Dimensionsgrößen und Zero-Filling der Eingangsdaten. Das Zero-Filling kann in jeder Dimension durchgeführt werden, z.B. als Zero-Filling einer 384x384-Bildmatrix für eine 512x512-Autoencoder-Eingangsschicht und/oder als Zero-Filling einer Aufnahme mit zwanzig (20) Schichten in eine Eingabeschicht, die auf vierzig (40) Schichten in der Extradimension trainiert wurde.

In einem dritten (mit dem ersten und/oder zweiten Ausführungsbeispiel kombinierbaren) Ausführungsbeispiel erfolgt eine Sliding-Window-Kodierung. Soll eine Schicht einer 20-Schichten-Aufnahme mit einem Autoencoder kodiert werden, dessen Eingangsschicht nur drei (3) Schichten in der Extradimension aufweist, so können die zwei Nachbarschichten mitverwendet werden. Die Extradimension der Ausgangsschicht des Decoders kann dann bei 1 liegen. Somit wird dennoch die Datenredundanz mit den Nachbarschichten berücksichtigt.

In einem vierten (mit dem ersten, zweiten und/oder dritten Ausführungsbeispiel kombinierbaren) Ausführungsbeispiel erfolgt eine Ausnutzung der hermiteschen k-Raum-Symmetrie. Die vier Quadranten des k-Raums können ggf. mittels Spiegelung und komplexer Konjugation in gleiche Orientierung gebracht und als Extradimension verwendet werden.

Alternativ oder ergänzend kann eine vierte Dimension in der Eingabeschicht aufgespannt und/oder unterschiedliche Dimensionen (z.B. für Spule und Schicht) in einer Extradimension aneinandergehängt (und/oder konkateniert) werden.

In einer weiteren Anwendungsform (die mit jedem vorhergehenden Ausführungsbeispiel kombinierbar ist) können nur Teile der Eingangsdaten kodiert werden, während anderen Teile unkomprimiert gespeichert und/oder übermittelt werden. Je nach Anwendungsfall kann z.B. nur das k-Raum-Zentrum, nur die k-Raum-Peripherie oder abwechselnd unterschiedliche Teile des k-Raums kodiert werden.

Weitere mögliche Anwendungen liegen in der Spulenkompression (hier kann oder muss u.U. die Kanalzahl des Decoders geringer gewählt werden als die des Encoders) und/oder in der Übertragung von Detektordaten bei der CT- oder PET-Bildgebung, insbesondere wenn die Übertragungsbandbreite begrenzt ist.

Die erfinderische Technik wendet einer Autoencoder-Netzwerk-Architektur zur Datenkompression unter Ausnutzung von Redundanz in digitalen medizinischen Bild-Datensätzen (z.B. umfassend MRT-Daten) an. Vorteilhafter wird dadurch ein geringerer Speicherplatzbedarf und/oder eine schnellere Datenübertragung ermöglicht.

Ein beispielhafter Anwendungsfall mittels der erfindungsgemäßen Technik liegt darin, die k-Raum-Daten, die nach der Bildrekonstruktion durch Fourier-Transformation aus den komplexen Bilddaten erzielt werden, zu komprimieren. Diese kann besonders sinnvoll sein für den Fall, dass rekonstruierte große Datensätze komprimiert gespeichert werden sollen, z.B. HCP-Aufnahmen. Die HCP-Aufnahmen umfassen insbesondere fMRI-Zeitserien. Bei den fMRI-Zeitserien kann es sinnvoll sein, direkt die komplexen Bilddaten zu verwenden und nicht zunächst in den k-Raum zurückzutransformieren.

Für die Anwendungsfälle, bei denen Daten schnell vom Scanner an einen Server zur Rekonstruktion übertragen werden sollen, liegen in der Regel nur k-Raum-Daten vor. Bilddaten können meistens noch nicht erzeugt werden, da dies erst im Rahmen der Rekonstruktion geschieht. Hier ist die direkte Verwendung der k-Raum-Daten bzw. der Hybridraum-Daten (insbesondere nach FT entlang der Ausleserichtung; diese FT kann in den meisten Fällen stattfinden, da diese Richtung meistens Nyquist-abgetastet vorliegt) für das Komprimieren mittels der erfindungsgemäßen Technik möglich.

Ein alternativer oder ergänzender Anwendungsfall für eine Kompression der Hybridraum-Daten mittels der erfindungsgemäßen Technik besteht für das sogenannte Readout Oversampling: In der Regel wird in Ausleserichtung ein mehr als zweimal so hohes Field Of View aufgenommen (wie insbesondere auf https://mriquestions.com/frequency-wrap-around.html beschrieben, dessen Inhalt hierin durch Bezugnahme aufgenommen ist). In den meisten Fällen, in denen in Ausleserichtung in diese Richtung keine Anteile der Körpers liegen (z.B. axiale Kopfbildgebung), enthalten diese Bereiche fast kein Signal, sind also in der Regel gut komprimierbar. Hier ist eine Kompression auch ohne Redundanzdimension möglich, wenn die Daten vor der Kompression entlang des Ausleserichtung per FT in den Hybridraum überführt werden.

In der nachfolgenden Tabelle sind beispielhaft unterschiedliche Datenredundanzdimensionen abhängig vom Anwendungsfall (und/oder Bildgebungsprotokoll) insbesondere für MRT aufgelistet.

| Beispielhafte Datenredundanzdimensionen pro Anwendungsfall, insbesondere für MRT | |
|---|---|
| Anwendungsfall | Dimension mit zu erwartender hoher Datenredundanz |
| Zeitserienaufnahmen (z.B. fMRI und/oder Kontrastmittel-Dynamik) | Zeitdimension |
| Konventionelle 2D-Aufnahme | Schichtdimension |
| Multi-Kontrast-Aufnahme (z.B. T1/T2/T2*-Mapping und/oder DIXON-Bildgebung) | Kontrastdimension |
| Alle Aufnahmen mit mehreren Spulenkanälen | Spulendimension |

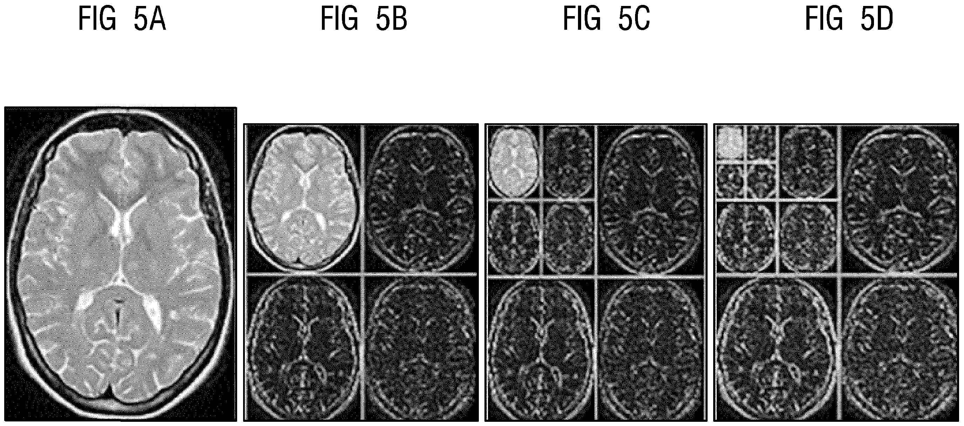

Fig. 5A, 5B, 5C und 5D zeigen ein Beispiel einer WT eines Kopfbildes in Reproduktion von Ali Alijamaat et al., "Multiple sclerosis identification in brain MRI images using wavelet convolutional neural networks", Int. J. Imaging Syst. Technol., Vol 31, No. 2, pages 778-785 (2021), dessen Inhalt hierin durch Bezugnahme umfasst ist. Fig. 5A zeigt das Originalbild, und Fig. 5B, 5C und 5D zeigen unterschiedliche WT-Ergebnisse bzw. Spektralzerlegungen des Bilds. Die schwarzen (und/oder homogenen) Bereiche nach der WT in Fig. 5B, 5C und 5D indizieren jeweils eine gute Komprimierbarkeit.

In einem weiteren Ausführungsbeispiel umfasst eine FT einer Herz-MRT-Zeitserie in Zeitrichtung. Dort, wo sich wenig im Bild ändert, finden sich nur Fourier-Koeffizienten mit niedriger Frequenz, der Rest ist annähernd Null. Somit ist eine Datenkomprimierung mittels der erfindungsgemäßen Technik nach FT in Zeitrichtung vorteilhaft.

Soweit nicht bereits explizit beschrieben, können einzelne Ausführungsformen oder deren einzelne Aspekte und Merkmale, die in Bezug auf die Zeichnungen beschrieben sind, miteinander kombiniert oder ausgetauscht werden, ohne den Umfang der beschriebenen Erfindung einzuschränken oder zu erweitern, wenn eine solche Kombination oder ein solcher Austausch sinnvoll und im Sinne der vorliegenden Erfindung ist. Vorteile, die in Bezug auf eine bestimmte Ausführungsform der vorliegenden Erfindung oder in Bezug auf eine bestimmte Figur beschrieben sind, sind, wo immer dies zutrifft, auch Vorteile anderer Ausführungsformen der vorliegenden Erfindung. Unabhängig vom grammatikalischen Geschlecht eines bestimmten Begriffes sind Personen mit männlicher, weiblicher oder anderer Geschlechteridentität mit umfasst.

## Patentansprüche

1. Computer-implementiertes Verfahren (100) zum Komprimieren eines digitalen medizinischen Bild-Datensatzes, der mittels eines medizinischen Scanners erhalten wurde, umfassend die Schritte:
- Empfangen (S102) einen Hinweis auf mindestens eine Datenredundanzdimension eines zu empfangenen (S104) digitalen medizinischen Bild-Datensatzes; und
- Empfangen (S104), an einer Eingangsschicht (302) eines trainierten Autoencoders (300), des digitalen medizinischen Bild-Datensatzes von dem medizinischen Scanner, wobei der trainierte Autoencoder (300) zumindest eine Extradimension (302-2; 308-2) in einer Eingangsschicht (304) und optional in einer Ausgangsschicht (310) umfasst, insbesondere wobei die zumindest eine Extradimension (302-2) in der Eingangsschicht (304) zum Empfangen von Daten im Hinblick auf die Datenredundanzdimension ausgebildet ist, vorzugsweise wobei die an der zumindest einen Extradimension (302-2) in der Eingangsschicht (304) empfangenen Daten zumindest einen, insbesondere im Hinblick auf die Datenredundanzdimension ausgewählten, Teil des empfangenen (S104) digitalen medizinischen Bild-Datensatzes umfassen; und
- Komprimieren (S106), mittels des trainierten Autoencoders (300), mindestens eines Teils des empfangenen (S104) digitalen medizinischen Bild-Datensatzes unter Verwendung des empfangenen (S102) Hinweises auf die mindestens eine Datenredundanzdimension und/oder unter Verwendung von an der zumindest einen Extradimension (302-2) empfangenen Daten.

2. Verfahren (100) gemäß Anspruch 1, ferner umfassend mindestens einen der Schritte:
- Übertragen (S108) des komprimierten (S106) digitalen medizinischen Bild-Datensatzes; und
- Speichern (S110) des komprimierten (S106) digitalen medizinischen Bild-Datensatzes.

3. Verfahren (100) gemäß einem der vorhergehenden Ansprüche, wobei der empfangene (S102) Hinweis auf die mindestens eine Datenredundanzdimension ausgewählt ist aus der Gruppe umfassend:
- Eine Information hinsichtlich einer Art einer Datenredundanz;
- Eine Auswahl des trainierten Autoencoders (300) aus einer Menge trainierter Autoencoder (300), wobei jeder Autoencoder (300) für eine vorbestimmte, insbesondere unterschiedliche, Art von Datenredundanz trainiert ist; und
- Eine Kopie zumindest eines Teils des digitalen medizinischen Bild-Datensatzes.

4. Verfahren (100) gemäß einem der vorhergehenden Ansprüche, wobei die zumindest eine Extradimension (308-2) in der Ausgangsschicht (310) dazu ausgebildet ist, einem Autodecoder eine Information hinsichtlich des verwendeten empfangenen (S102) Hinweises im Schritt des Komprimierens (S106) bereitzustellen.

5. Verfahren (100) gemäß einem der vorhergehenden Ansprüche, ferner umfassend den Schritt:
- Bestimmen (S103) eines Kompressionsfaktors für das Komprimieren (S106) des mindestens einen Teils des empfangenen (S104) digitalen medizinischen Bild-Datensatzes.

6. Verfahren (100) gemäß einem der vorhergehenden Ansprüche, wobei der empfangene (S104) digitale medizinische Bild-Datensatz mindestens eine der folgenden Datenarten umfasst:
- Rohdaten einer Aufnahme des medizinischen Scanners;
- Daten in einem transformierten Raum;
- Daten im k-Raum einer Magnetresonanztomographie, MRT, -Aufnahme;
- Daten im, insbesondere komplexen, Bildraum einer Aufnahme des medizinischen Scanners; und
- Daten in einem Hybridraum einer MRT-Aufnahme.

7. Verfahren (100) gemäß einem der vorhergehenden Ansprüche, wobei die Datenredundanzdimension mindestens eine der folgenden Dimensionen umfasst:
- Eine Zeitdimension im Fall, dass der digitale medizinische Bild-Datensatz eine Zeitserienaufnahme umfasst;
- Eine Repetitionsdimension im Fall, dass der digitale medizinische Bild-Datensatz eine Anzahl Repetitionen umfasst;
- Eine Kontrastdimension im Fall, dass der digitale medizinische Bild-Datensatz eine Multi-Kontrastaufnahme umfasst;
- Eine Kanaldimension im Fall, dass der digitale medizinische Bild-Datensatz eine Mehrkanalaufnahme umfasst:
- Eine Schichtdimension im Fall, dass der digitale medizinische Bild-Datensatz eine mehrschichtige Aufnahme umfasst; und
- Eine Spulendimension im Fall, dass der digitale medizinische Bild-Datensatz eine Aufnahme mit mehreren Spulen umfasst.

8. Verfahren (100) gemäß einem der vorhergehenden Ansprüche, wobei der digitale medizinische Bild-Datensatz von dem medizinischen Scanner gemäß einem vorbestimmtem Bildgebungsprotokoll erhalten wird.

9. Verfahren (100) gemäß einem der vorhergehenden Ansprüche, wobei der mindestens eine komprimierte (S106) Teil des digitalen medizinischen Bild-Datensatzes einen Bereich im Bildraum, insbesondere ein Zentrum und/oder eine Peripherie umfasst; und/oder
wobei der medizinische Scanner einen MRT umfasst und wobei der mindestens eine komprimierte (S106) Teil des digitalen medizinischen Bild-Datensatzes ein k-Raum-Zentrum, eine k-Raum-Peripherie und/oder sich abwechselnde unterschiedliche Teile des k-Raums umfasst.

10. Verfahren (100) gemäß einem der vorhergehenden Ansprüche, wobei das Komprimieren (S106) des mindestens einen Teils des empfangenen (S104) digitalen medizinischen Bild-Datensatzes ferner basiert auf und/oder der empfangene (S102) Hinweis auf die mindestens eine Datenredundanzdimension umfasst:
- Einer Sliding-Window-Kodierung benachbarter Schichten innerhalb des digitalen medizinischen Bild-Datensatzes und/oder benachbarter zeitlicher Instanzen einer Zeitserienaufnahme innerhalb des digitalen medizinischen Bild-Datensatzes;
- Einer Symmetrieeigenschaft des digitalen medizinischen Bild-Datensatzes, insbesondere einer hermiteschen k-Raum-Symmetrie einer MRT-Aufnahme; und/oder
- Einer Überabtastung entlang einer Ausleserichtung, insbesondere für einen digitalen medizinischen Bild-Datensatz umfassend Daten im Hybridraum einer MRT-Aufnahme.

11. Verfahren (100) gemäß einem der vorhergehenden Ansprüche, wobei der Autoencoder (300) auf die Datenredundanzdimension trainiert wird, umfassend die Schritte:
- Empfangen (S202), an der Eingangsschicht (304; 302-2) des Autoencoders (300) und insbesondere an zumindest einer Extradimension (302-2) der Eingangsschicht (304), eines Hinweises auf mindestens eine Datenredundanzdimension eines digitalen medizinischen Trainings-Bild-Datensatzes, wobei der digitale medizinische Trainings-Bild-Datensatz insbesondere mittels eines medizinischen Scanners erhalten wurde;
- Empfangen (S204), an der Eingangsschicht (304; 302-1) des Autoencoders (300), des digitalen medizinischen Trainings-Bild-Datensatzes, insbesondere Empfangen von Daten im Hinblick auf die Datenredundanzdimension an der zumindest einen Extradimension (302-2) der Eingangsschicht (304), vorzugsweise wobei die an der zumindest einen Extradimension (302-2) in der Eingangsschicht (304) empfangenen Daten zumindest einen, insbesondere im Hinblick auf die Datenredundanzdimension ausgewählten, Teil des empfangenen (S204) digitalen medizinischen Trainings-Bild-Datensatzes umfassen;
- Komprimieren (S206), mittels verborgener Schichten (306) des Autoencoders (300), mindestens eines Teils des empfangenen (S204) digitalen medizinischen Test-Bild-Datensatzes, insbesondere unter Verwendung des empfangenen (S202) Hinweises auf die mindestens eine Datenredundanzdimension des digitalen medizinischen Trainings-Bild-Datensatzes und/oder unter Verwendung von an der zumindest einen Extradimension (302-2) empfangenen Daten;
- Ausgeben (S208), von einer Ausgangsschicht (310) des Autoencoders (300) an eine Eingangsschicht eines Autodecoders, des Ergebnisses des Komprimierens (S208) des mindestens einen Teils des empfangenen (S204) digitalen medizinischen Test-Bild-Datensatzes;
- Dekomprimieren (S210), mittels verborgener Schichten des Autodecoders, des Ergebnisses des Komprimierens (S208) des mindestens einen Teils des empfangenen (S204) digitalen medizinischen Test-Bild-Datensatzes;
- Ausgeben (S212) des Ergebnisses des Dekomprimierens (S210) und Anwenden (S214) einer Verlustfunktion auf den empfangenen (S204) digitalen medizinischen Trainings-Bild-Datensatz und auf das ausgegebene (S212) Ergebnis des Dekomprimierens (S210);
wobei zumindest die Schritte des Komprimierens (S206) durch den Autoencoder (300), des Ausgebens (S208) vom Autoencoder (300) zum Autodecoder und des Dekomprimierens (S210) durch den Autodecoder wiederholt werden, um die angewendete (S214) Verlustfunktion zu optimieren.

12. Verfahren (100) gemäß dem direkt vorhergehenden Anspruch, ferner umfassend eine Rückpropagation und/oder ein Gradientenverfahren zum Trainieren des Autoencoders (300).

13. Verfahren (100) gemäß einem der vorhergehenden Ansprüche, wobei der Autoencoder (300) groß dimensioniert ist und/oder eine Möglichkeit zum Weglassen von Eingangsdaten aufweist, insbesondere abhängig von einer Art von Datenredundanzdimension und digitalem medizinischen Trainings-Bild-Datensatz, wobei der Autoencoder (300) mit einer Mehrzahl unterschiedlicher Arten von digitalen medizinischen Trainings-Bild-Datensätzen und Datenredundanzdimensionen trainiert wird.

14. Trainiertes Autoencoder-Netzwerk (300) zum Komprimieren eines digitalen medizinischen Bild-Datensatzes, der mittels eines medizinischen Scanners erhalten wurde,
- wobei eine Eingangsschicht (304; 302-1) des trainierten Autoencoder-Netzwerks (300) zum Empfangen des digitalen medizinischen Bild-Datensatzes ausgebildet ist;
- wobei die Eingangsschicht (304) des trainierten Autoencoder-Netzwerks (300) zumindest eine Extradimension (302-2) umfasst, wobei die zumindest eine Extradimension (302-2) dazu ausgebildet ist, einen Hinweis auf mindestens eine Datenredundanzdimension des empfangenen digitalen medizinischen Bild-Datensatzes zu empfangen, und/oder insbesondere wobei die zumindest eine Extradimension (302-2) in der Eingangsschicht (304) zum Empfangen von Daten im Hinblick auf die Datenredundanzdimension ausgebildet ist, vorzugsweise wobei die an der zumindest einen Extradimension (302-2) in der Eingangsschicht (304) empfangenen Daten zumindest einen, insbesondere im Hinblick auf die Datenredundanzdimension ausgewählten, Teil des empfangenen digitalen medizinischen Bild-Datensatzes umfassen;
- wobei das trainierte Autoencoder-Netzwerk (300) zum Komprimieren mindestens eines Teils des empfangenen digitalen medizinischen Bild-Datensatzes unter Verwendung des empfangenen Hinweises auf die mindestens eine Datenredundanzdimension und/oder unter Verwendung von an der zumindest einen Extradimension (302-2) empfangenen Daten ausgebildet ist; und
- wobei eine Ausgangsschicht (310) des trainierten Autoencoder-Netzwerks (300) optional zumindest eine in Antwort auf einen empfangenen Hinweis auf mindestens eine Datenredundanzdimension des empfangenen digitalen medizinischen Bild-Datensatzes bereitgestellte Extradimension (308-2) umfasst, wobei die in der Ausgangsschicht (310) bereitgestellte zumindest eine Extradimension (308-2) vorzugsweise einen Hinweis auf die zum Komprimieren verwendete Datenredundanzdimension umfasst und/oder bereitstellt.

15. Autoencoder-Netzwerk (300) gemäß dem direkt vorhergehenden Anspruch, wobei das Autoencoder-Netzwerk (300) ferner zum Ausführen des Verfahrens (100) gemäß Ansprüchen 2 bis 10 oder 13 ausgebildet ist, und/oder wobei das Autoencoder-Netzwerk (300) gemäß dem Verfahren (100) nach Anspruch 11 oder 12 trainiert ist.

16. System für zum Komprimieren eines digitalen medizinischen Bild-Datensatzes, der mittels eines medizinischen Scanners erhalten wurde, umfassend:
- eine Scannerschnittstelle zu mindestens einem medizinischen Scanner; und
- zumindest ein Autoencoder-Netzwerk (300) gemäß Anspruch 14 oder 15.

17. Computerprogrammprodukt mit Programmelementen, die einen Autoencoder-Netzwerk (300) veranlassen, die Schritte des Verfahrens (100; 200) zum Komprimieren eines digitalen medizinischen Bild-Datensatzes, der mittels eines medizinischen Scanners erhalten wurde, und/oder zum Trainieren des Autoencoder-Netzwerks (300) nach einem der vorhergehenden Verfahrensansprüche auszuführen, wenn die Programmelemente in einen Speicher des Autoencoder-Netzwerk (300) geladen werden.
